(19) Europäisches Patentamt
European Patent Office
Office européen des brevets

(11) Veröffentlichungsnummer : **0 116 276**
**B1**

(12) EUROPÄISCHE PATENTSCHRIFT

(45) Veröffentlichungstag der Patentschrift : 11.01.89

(21) Anmeldenummer : 84100130.8

(22) Anmeldetag : 09.01.84

(51) Int. Cl.⁴ : C 07 K 5/06, A 61 K 37/02, C 07 D209/96

ERRATUM

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE,COLONNE,LIGNE) | | LAUTET BERICHTIGT:<br>SHOULD READ :<br>DEVRAIT ETRE LU : |
|---|---|---|---|
| monosubstituted, or trisubstituted | 23 | 44 | monosubstituted, disubstituted or trisubstituted |

Tag der Entscheidung über die Berichtigung
Date of decision on rectification: 03.02.89

Ausgabe- und Veröffentlichungstag:
Issue and publication date:
Date d'edition et de ... : 05.04.89

Patbl.Nr.
EPB no: 89/1

(19) Europäisches Patentamt
European Patent Office
Office européen des brevets

(11) Veröffentlichungsnummer : **0 116 276 B1**

(12) # EUROPÄISCHE PATENTSCHRIFT

(45) Veröffentlichungstag der Patentschrift : **11.01.89**

(51) Int. Cl.⁴ : **C 07 K 5/06**, A 61 K 37/02, C 07 D209/96

(21) Anmeldenummer : **84100130.8**

(22) Anmeldetag : **09.01.84**

(54) **Neue spirocyclische Aminosäurederivate, Verfahren zu ihrer Herstellung, diese enthaltende Mittel und deren Verwendung sowie neue spirocyclische Aminosäuren als Zwischenprodukte und Verfahren zu deren Herstellung.**

(30) Priorität : **12.01.83 DE 3300774**

(43) Veröffentlichungstag der Anmeldung : **22.08.84 Patentblatt 84/34**

(45) Bekanntmachung des Hinweises auf die Patenterteilung : **11.01.89 Patentblatt 89/02**

(84) Benannte Vertragsstaaten : **AT BE CH DE FR GB IT LI LU NL SE**

(56) Entgegenhaltungen :
**EP-A- 0 012 401**
**EP-A- 0 050 800**
**EP-A- 0 090 341**

(73) Patentinhaber : **HOECHST AKTIENGESELLSCHAFT**
**Postfach 80 03 20**
**D-6230 Frankfurt am Main 80 (DE)**

(72) Erfinder : **Henning, Rainer, Dr.**
**Rotenhofstrasse 31**
**D-6234 Hattersheim am Main (DE)**
Erfinder : **Urbach, Hansjörg, Dr.**
**Le Lavandoustrasse 41**
**D-6242 Kronberg/Taunus (DE)**
Erfinder : **Teetz, Volker, Dr.**
**An der Tann 20**
**D-6238 Hofheim am Taunus (DE)**
Erfinder : **Becker, Reinhard, Dr.**
**Adelheidstrasse 101**
**D-6200 Wiesbaden (DE)**

**EP 0 116 276 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aus der EP-A-12 401 sind u. a. von Prolin abgeleitete Carboxyalkyldipeptid-Derivate bekannt. Die EP-A-50 800 betrifft u. a. mono- und bicyclische Carboxyalkyldipeptid-Derivate. In den Dokumenten werden außerdem Verfahren zur Herstellung der genannten Verbindungen, diese enthaltende Zubereitungen und ihre Verwendung als Angiotensin-Converting-Enzyme-Inhibitoren beschrieben.

Die Erfindung betrifft neue spirocyclische Aminosäure-derivate der Formel I

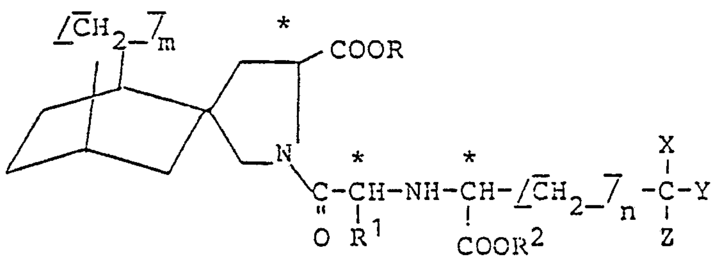

(I)

in der

m = 1 oder 2,

n = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino insbesondere ($C_1$ bis $C_6$)-Alkanoylamino oder BOC—NH, oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_5$ bis $C_{12}$)-Aryl-($C_1$ bis $C_4$)-alkyl oder ($C_7$ bis $C_{13}$)-Aroyl-($C_1$ bis $C_2$)-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, oder eine gegebenenfalls geschützte Seitenkette einer natürlich vorkommenden α-Aminosäure,

$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$-$C_4$)-alkylamino und/oder Methylendioxy mono-, di- oder trisubstituiert sein kann, oder 3-Indolyl bedeuten, sowie deren physiologisch unbedenkliche Salze.

Falls $R^1$ für eine geschützte Seitenkette einer natürlich vorkommenden α-Aminosäure steht, wie z. B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Aminoschutzgruppen, insbesondere aber ($C_1$-$C_6$)-Alkanoyl bevorzugt. Im Falle, daß $R^1$ die geschützte Tyrosin-Seitenkette bedeutet, wird die Ether-Schutzgruppe am Sauerstoff, insbesondere ($C_1$-$C_6$)-Alkyl, bevorzugt ; besonders bevorzugte Schutzgruppen sind Methyl und Ethyl.

Als Salze kommen insbesondere Alkali- und Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Unter Aryl ist hier wie im folgenden vorzugsweise gegebenenfalls substituiertes Phenyl oder Naphthyl zu verstehen. Unter Aroyl wird insbesondere Benzoyl verstanden. Alkyl kann geradkettig oder verzweigt sein.

Unter einem mono- bzw. bicyclischer Heterocylen-Rest mit 5 bis 7 bzw. 8 bis 10 Ringatomen, wovon 1 bis 4 Ringatome Stickstoffatome und/oder wovon 1 bis 4 Ringatome Stickstoffatome darstellen, wird beispielsweise Thienyl, Benzo [b] thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Pyrinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Verbindungen der Formel I besitzen chirale C-Atome. Sowohl die R- als auch die S-Konfigurationen an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen die mit einem Stern (*) markierten C-Atome S-Konfiguration aufweisen. Falls (—CO—CHR$^1$—NH—) = Cys ist, wird jedoch die R-Konfiguration dieses Zentrums bevorzugt.

Besonders bevorzugte Verbindungen der Formel I sind solche, in denen
m = 1 oder 2,
n = 1,
R = Wasserstoff oder Alkyl mit 1 bis 4 C-Atomen
$R_1$ = Wasserstoff, ($C_1$ bis $C_3$)-Alkyl, ($C_2$ oder $C_3$)-Alkenyl, Benzyl, 4-Methoxybenzyl, 4-Ethoxybenzyl, Phenethyl, 4-Amino-butyl oder Benzoylmethyl,
$R_2$ = Wasserstoff, ($C_1$ bis $C_4$)-Alkyl oder Benzyl und
X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$) alkyl-amino, Nitro und/oder Methylendioxy mono- oder disubstituiert oder im Falle von Methoxy, trisubstituiert sein kann, bedeuten, insbesondere solche Verbindungen der Formel I, in denen m = 1 oder 2, n = 1, R = Wasserstoff, $R^1$ = Methyl, 4-Methoxybenzyl oder 4-Ethoxybenzyl, $R^2$ = Wasserstoff oder Ethyl bedeuten und die chiralen C-Atome, die mit einem Stern (*) gekennzeichnet sind, die S-Konfiguration besitzen.

Im tricyclischen Aminosäurerest kann die —COOR-Gruppe exo- oder endo-ständig zum bicyclischen Reste stehen ; die bevorzugten Verbindungen der Erfindung beinhalten also eine der beiden Teilstrukturen der Formel Ia und Ib

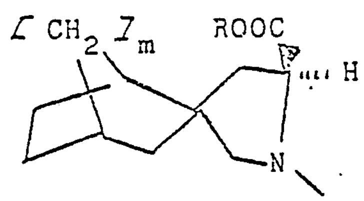

Ia (endo-Konfiguration)

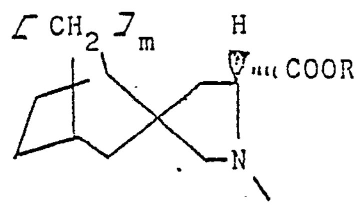

Ib (exo-Konfiguration)

sowie deren Spiegelbilder.

Die Erfindung betrifft ferner Verfahren zur Herstellung von Verbindungen der Formel I. Eine Verfahrensvariante ist dadurch gekennzeichnet, daß man eine Verbindung der Formel II,

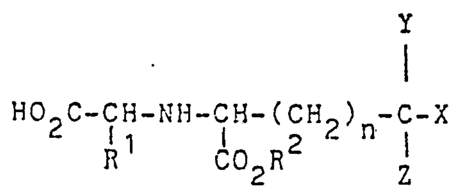

(II)

worin n, $R^1$, $R^2$, X, Y und Z die Bedeutung wie in Formel I haben mit einer Verbindung der Formel III,

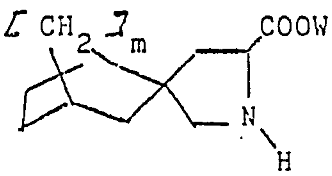

(III)

in welcher W = Wasserstoff oder einen sauer oder hydrogenolytisch abspaltbaren Rest, insbesondere einen tert.-Butyl-Rest oder einen Benzylrest bedeutet, nach bekannten Amidbildungsmethoden der Peptidchemie umsetzt und gegebenenfalls anschließend durch Säurebehandlung oder Hydrierung den Rest W und gegebenenfalls durch zusätzliche Säure- oder Basenbehandlung auch den Rest $R^2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV

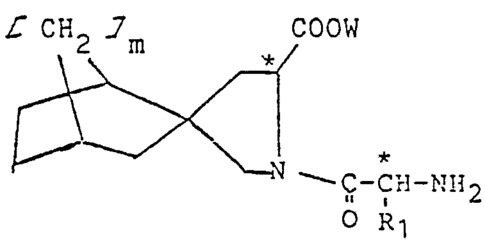

(IV)

in welcher m und $R^1$ die Bedeutung wie in Formel I und W die Bedeutung wie in Formel III besitzen, mit einer Verbindung der Formel V

$$R^2O_2C—CH=CH—CO—X \quad (V)$$

worin $R^2$ und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest $R^2$, wie oben beschrieben, abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$$OHC—CO_2R^2 \quad (VI) \qquad X—CO—CH_3 \quad (VII)$$

worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest $R^2$ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem. Soc. 93, 2897 (1971) beschriebenen Verfahrensweise mit einer Verbindung der Formel VIII

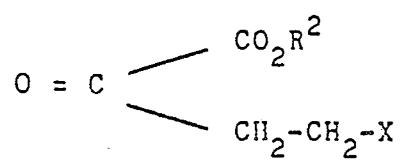

(VIII)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann elektrolytisch oder mit Reduktionsmittel wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisen erhaltenen Verbindung I mit Y und Z = zusammen Sauerstoff erhalten werden. Diese Reduktion kann mit einem Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$ bis $C_9$)-Aralkyl bedeutet, überführt werden.

Die Erfindung betrifft auch Verbindungen der Formel III

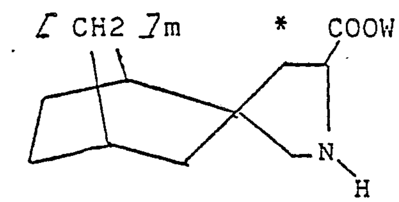

(III)

worin m = 1 oder 2 und W = Wasserstoff oder einen sauer oder hydrogenolytisch abspaltbaren Rest wie tert.-Butyl oder Benzyl bedeutet. Diese Verbindungen dienen gemäß der Erfindung als Ausgangsstoffe bei der Synthese von Verbindungen der Formel I und können erfindungsgemäß dadurch hergestellt werden, daß man

eine Verbindung der Formel XIV

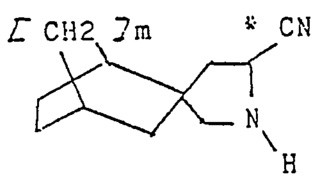

(XIV)

mit einer Mineralsäure wie Salzsäure oder Bromwasserstoffsäure oder mit einer starken Base wie einem Alkalihydroxid bei 20 °C bis 150 °C, insbesondere bei 60 bis 120 °C vorzugsweise in Wasser als

Lösungsmittel verseift und die erhaltenen Aminosäuren (III/W = Wasserstoff) gegebenenfalls nach üblichen Methoden der Aminosäurechemie verestert. Die Aminosäuren (III/W = Wasserstoff) bzw. die erhaltenen Ester (III/W $\neq$ Wasserstoff) können z. B. durch Chromatographie oder fraktionierte Kristallisation geeigneter Salze in Steroisomere auftrennt werden.

Die tricyclischen Nitrile der Formel XIV können erfindungsgemäß dadurch hergestellt werden, daß man ein bicyclisches Nitril der Formel (IX)

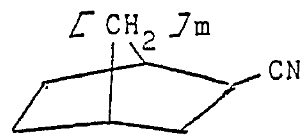

(IX)

in der m = 1 oder 2 ist, in Gegenwart einer starken Base, wie einem Alkaliamid, bevorzugt Lithiumdiisopropylamid, Lithiumdiethylamid oder Lithiumhexamethyldisilazid mit einer Verbindung der Formel (X)

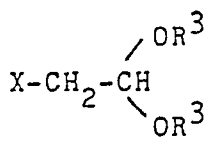

(X)

worin X Halogen, besonders Chlor, Brom oder Jod und $R^3$ gleiche oder verschiedene ($C_1$ bis $C_4$)-Alkylgruppen bedeuten oder beide Reste $R^3$ zusammen eine Ethylen- oder Propylenbrücke bilden, in einem aprotischen Lösungsmittel wie Tetrahydrofuran bei —100 °C bis + 50 °C, vorzugsweise —80 °C bis 0 °C zu einer Verbindung der Formel (XI)

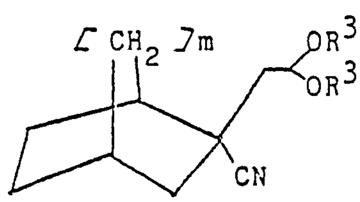

(XI)

in der m und $R^3$ die vorstehende Bedeutungen haben, umsetzt, diese mit einem geeigneten Reduktionsmittel wie z. B. Lithiumaluminiumhydrid in einem aprotischen Lösungsmittel, vorzugsweise Diethylether oder Tetrahydrofuran bei 0 °C bis 80 °C, vorzugsweise bei 20 °C bis 60 °C oder mit einem alkoholischen Lösungsmittel, vorzugsweise Ethanol oder n-Butanol bei 0 °C bis 120 °C, vorzugsweise bei 20 °C bis 80 °C oder durch katalytische Hydrierung in einem alkoholischen Lösungsmittel unter Zusatz von Ammoniak bei 0 °C bis 80 °C, vorzugsweise 20 °C bis 50 °C in Gegenwart eines Edelmetall- oder Ni-Katalysators, vorzugsweise Raney-Nickel oder Rhodium auf Aluminiumoxid zu einer Verbindung der Formel (XII)

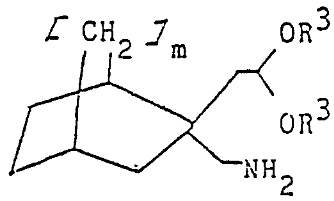

(XII)

in der m und $R^3$ die vorstehenden Bedeutungen haben, reduziert, diese durch Behandlung mit einer Mineralsäure, vorzugsweise Chlorwasserstoffsäure in Wasser bei 20 °C bis 140 °C, vorzugsweise 40 °C bis 100 °C zu einer Verbindung der Formel (XIII)

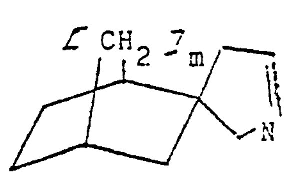

(XIII)

in der m = 1 oder 2 ist und die im Gemisch mit ihrem Trimeren anfällt cyclisiert, diese durch Umsetzung

mit einem Alkalicyanid, vorzugsweise Natrium- oder Kaliumcyanid in Wasser unter Zusatz einer Mineralsäure, vorzugsweise Salzsäure oder Schwefelsäure bei 0 °C bis 60 °C vorzugsweise bei 10 °C bis 40 °C in das Nitril der Formel (XIV) überführt.

Die Nitrile der Formel IX sind bekannt.

Die als Ausgangsstoffe zur Herstellung der Verbindungen der Formel I verwendeten Verbindungen der Formel II mit n = 1, Y, Z = Wasserstoff, $R^1$ = Methyl und $R^2$ = Methyl oder Ethyl und X = Phenyl sind bekannt (EP-A-37 231). Die Verbindungen der Formel II lassen sich nach verschiedenen Verfahrensweisen herstellen. Eine Synthesevariante geht von einem Keton der oben genannten Formel VII aus, das nach bekannten Verfahrensweisen in einer Mannich-Reaktion mit einer Verbindung der oben genannten Formel VI zusammen mit Aminosäureestern der Formel XV

$$H_2N - \underset{R^1}{\underset{|}{CH}} - CO_2W' \qquad \text{(XV)}$$

$$W'O_2C-\underset{R^1}{\underset{|}{\overset{*}{C}H}}-NH-\underset{CO_2R^2}{\underset{|}{\overset{*}{C}H}}-CH_2-CO-X \qquad \text{(XVI)}$$

worin $R^1$ die oben genannte Bedeutung besitzt und W' einen hydrogenolytisch oder sauer abspaltbaren Rest, insbesondere einen Benzyl- oder einen tert.-Butyl-Rest, bedeutet, zu einer Verbindung der Formel XVI, worin $R^1$, $R^2$, X und W' die oben genannten Bedeutungen besitzen, mit der Einschränkung, daß, wenn W' einen hydrogenolytisch abspaltbaren Rest, insbesondere Benzyl, bedeutet, $R^2$ nicht die Bedeutung von W' besitzen darf, umsetzt. Spaltet man den Rest W' hydrogenolytisch mit Hilfe von beispielsweise Palladium ab, werden bei einer Wasserstoffaufnahme von 3 Moläquivalenten Verbindungen der Formel II mit X, Z = Wasserstoff erhalten. Stoppt man die Wasserstoffaufnahme bei 1 Moläquivalent, erhält man Verbindungen der Formel II mit n = 1 und Y und Z zusammen = Sauerstoff, die man ebenfalls erhält, wenn der Rest W' der Formel XVI mit Säuren, wie beispielsweise Trifluoressigsäure oder Salzsäure, in einem inerten organischen Lösungsmittel, wie beispielsweise Dioxan, abgespalten wird.

Verbindungen der Formel XVI sind auch durch Michael-Additionen einer Verbindung der obengenannten Formel V mit einer Verbindung der obengenannten Formel XV nach bekannten Verfahrensweisen zugänglich. Bevorzugt eignet sich dieses Verfahren zur Herstellung von solchen Verbindungen der Formel XVI, in denen $R^1$ = Methyl, $R^2$ = Ethyl und X = Aryl bedeuten.

Die Verbindungen der Formel XVI fallen als Diastereomerengemische an. Bevorzugte Diastereomere der Formel XVI sind solche, in denen die mit einem Stern markierten chiralen C-Atome jeweils S-Konfiguration aufweisen. Diese können beispielsweise durch Kristallisation oder durch Chromatographie, z. B. an Kieselgel, abgetrennt werden. Bei der nachfolgenden Abspaltung des Restes W' bleiben die Konfigurationen der chiralen C-Atome erhalten.

Die als Ausgangsstoffe zur Herstellung der Verbindungen der Formel I verwendeten Verbindungen der obengenannten Formel IV werden aus den Verbindungen der obengenannten Formel III durch Umsetzen mit einer N-geschützen 2-Aminocarbonsäure der Formel XVII

$$V - HN - \underset{R^1}{\underset{|}{CH}} - CO_2H \qquad \text{(XVII)}$$

worin V eine Schutzgruppe ist und $R^1$ die obengenannte Bedeutung besitzt, nach bekannten Verfahrensweise erhalten. Als Schutzgruppe V, die nach beendeter Reaktion wieder abspalten wird, kommt beispielsweise tert.-Butoxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden sauren Abspaltung des Restes W werden als Säuren bevorzugt Trifluoressigsäure oder Chlorwasserstoff eingesetzt.

Die gemäß oben beschriebener Verfahrensweise erhaltenen Verbindungen der Formel III fallen als Gemisch an und können beispielsweise durch Umkristallisieren oder durch Chromatographie voneinander getrennt werden.

Die Verbindungen der Formel III fallen als racemische Gemische an und können als solche in die weiteren oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantionmere eingesetzt werden.

Fallen die Verbindungen der Formel I als Racemate an, können auch diese nach den üblichen Methoden wie beispielsweise über Salzbildung mit optisch aktiven Basen oder Säuren in ihre

Enantiomeren gespalten oder durch Chromatographie getrennt werden.

Die erfindungsgemäßen Verbindungen der Formel I, liegen falls R = Wasserstoff ist, als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z. B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 0,01-7 mg/kg/Tag, insbesondere bei 0,07-0,5 mg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z. B. in Frage : Wasser, physiologische Kochsalzlösungen oder Alkohole, z. B. Ethanol, Propandiol, oder Glycerin, daneben auch Zuckerlösungen wie Glukose- oder Mannitlösungen, oder auch eine Mischung aus den verschiedenen genannten Lösungsmitteln.

Die außerordentlich starke Wirksamkeit der Verbindungen gemäß Formel I wird durch die pharmakologischen Daten der nachfolgenden Tabelle belegt :

Intraduodenale Gabe an der narkotisierten Ratte, 50 % Hemmung der durch 310 ng Angiotensin I ausgelösten Pressorreaktion 30 min. nach Applikation in der Dosis ... = $ED_{50}$ :

Tabelle

(Die aufgeführten Verbindungen besitzen die S-Konfiguration an den mit einem Stern gekennzeichneten C-Atomen) :

| m | n | X | Y | Z | $R^1$ | $R^2$ | R | Konfiguration der COOR-Gruppe | ED (µg/kg) |
|---|---|---|---|---|---|---|---|---|---|
| 2 | 1 | $C_6H_5$ | H | H | $CH_3$ | $C_2H_5$ | H | exo | 50 |
| 2 | 1 | $C_6H_5$ | H | H | $CH_3$ | $C_2H_5$ | H | endo | 500 |
| 2 | 1 | $C_6H_5$ | H | H | $CH_3$ | H | H | exo | 200 |
| 2 | 1 | $C_6H_5$ | H | H | $CH_3$ | H | H | endo | 850 |
| 2 | 1 | $C_6H_5$ | - O - | | $CH_3$ | $C_2H_5$ | H | exo | 80 |
| 2 | 1 | $C_6H_5$ | - O - | | $CH_3$ | $C_2H_5$ | H | endo | 650 |
| 1 | 1 | $C_6H_5$ | H | H | $CH_3$ | $C_2H_5$ | H | exo/endo | 80 |
| 1 | 1 | $C_6H_5$ | H | H | $CH_3$ | H | H | exo/endo | 240 |
| 1 | 1 | $C_6H_5$ | - O - | | $CH_3$ | $C_2H_5$ | H | exo/endo | 140 |

Die Symbole n, m, X, Y, Z, R, $R^1$ und $R^2$ beziehen sich auf die Verbindungen der Formel I.

Die nachfolgenden Beispiele dienen zur Erläuterung der Erfindung, ohne diese auf die stellvertretend genannten Verbindungen zu beschränken.

Beispiel 1

5'-Cyano-spiro [bicyclo [2.2.2] octan-2,3'-pyrrolidin]

a) 2-Cyano-2-(1,3-dioxolan-2-yl)-methyl-bicyclo [2.2.2] octan

84 ml Diisopropylamin werden in 300 ml absoluten Tetrahydrofuran gelöst. Unter trockenem Argon gibt man bei — 20 °C 39,9 ml n-Butyllithium (1,5-Molar in Hexan) zu. Nach 30 Minuten bei Raumtemperatur wird auf — 78 °C abgekühlt und 8,1 g 2-Cyano-bicyclo [2.2.2] octan in 30 ml absolutem Tetrahydrofuran zugerührt, nach 30 Minuten bei 0 °C erneut auf — 78 °C abgekühlt und 10 g 2-Brommethyl-1,3-dioxolan in 30 ml Tetrahydrofuran zugetropft. Man rührt noch 45 Minuten bei — 78 °C, wärmt auf Raumtemperatur auf, engt ein, nimmt den Rückstand mit 2 N Essigsäure und Ether auf, wäscht dreimal mit Wasser, trocknet über Natriumsulfat und entfernt das Lösungsmittel. Das Rohprodukt wird an Kieselgel mit Essigester/Cyclohexan (1 : 6) als Laufmittel chromatographiert. Man erhält 7,9 g der Titelverbindung als farbloses Öl.

$^1$H-NMR-Daten ($CDCl_3$) :
5,02 + 4,95 (2t, J = 6 Hz, 1H) ;
4,1-3,7 (m, 4H) ;
1,95 (d, J = 6 Hz, 2H) ;
2,2-1,3 (m, 12H) ppm

b) 2-Aminomethyl-2-(1,3-dioxolan-2-yl)-methyl-bicyclo [2.2.2] octan

3,7 g Lithiumaluminiumhydrid werden in 60 ml trockenen Ether suspendiert ; 7,4 g 2-Cyano-2-(1,3-dioxolan-2-yl)-methyl-bicyclo [2.2.2] octan werden gelöst in 80 ml trockenem Ether so zugetropft, daß leichtes Sieden aufrechterhalten wird. Anschließend wird 2 Stunden am Rückfluß gekocht, dann unter Eiskühlung tropfenweise nacheinander mit 2,1 ml Wasser, 2,1 ml 1 N Natronlauge und 15 ml Wasser versetzt. Das Aluminiumhydroxid wird, abgesaugt und gut mit Ether nachgewaschen. Man erhält 5,5 g der Titelverbindung als farbloses Öl.

$^1$H-NMR-Daten ($CDCl_3$) :
4,87 (t, J = 6 Hz, 1H) ;
4,0-3,6 (m, 4H) ;
2,63 (s, 2H) ;
2,0-1,0 (m, 14H) ppm

c) Spiro [bicyclo [2.2.2] octan-2,3'-pyrrolin-$\Delta^{1'}$

5,5 g 2-Aminomethyl-2-(1,3-dioxolan-2-yl)-methyl-bicyclo [2.2.2] octan werden mit 5 N Salzsäure 1,5 Stunden am Rückfluß gekocht. Nach Extraktion mit Essigester wird die wäßrige Phase unter Eiskühlung mit 4 N Kaliumhydroxid versetzt, mit Ether extrahiert, der Extrakt mit Natriumsulfat getrocknet und das Lösungsmittel entfernt. Man erhält 3 g der Titelverbindung als Gemisch des Monomeren und des Trimeren (s-Triazin-Derivat).

d) 5'-Cyano-spiro [bicyclo [2.2.2] octan-2,3'-pyrrolidin]

3 g Spiro [bicyclo [2.2.2] octan-2,3'-pyrrolin-$\Delta^{1'}$ werden zusammen mit 2,25 g Kaliumcyanid in 100 ml Wasser gelöst. Unter Eiskühlung werden 24 ml 2 N Salzsäure zugetropft und 72 Stunden bei Raumtemperatur gerührt. Nach Extraktion mit Essigester wird die wäßrige Lösung mit 1 N Natronlauge alkalisch gestellt, mit Ether extrahiert und der Extrakt über Natriumsulfat getrocknet. Nach Einengen wird das Rohprodukt an Kieselgel mit Essigester/Cyclohexan (1 : 1) als Laufmittel chromatographiert, wodurch die endo- und exo-Isomeren getrennt werden.
endo-Isomer : 0,83 g, Schmp. 78-80 °C

$^1$H-NMR-Daten ($CDCl_3$) :
4,0 (t, J = 14 Hz, 1H) ;
2,8 (s, 2H) ;
2,4 (s, 1H) ;
2,0 (d, 2H) ;
1,5 (br. s, 12H) ;

exo-Isomer : 1,5 g, Schmp. 38-40 °C

$^1$H-NMR-Daten ($CDCl_3$) :
4,02 (x-Teil eines ABX-Systems, 1H) ;
2,85 (AB-System, J = 15 Hz, 2H) ;
2,5-1,0 (m, 15H) ppm.

Beispiel 2

Endo-Spiro [bicyclo [2.2.2] octan-2,3'-pyrrolidin]-5'-yl-carbonsäure

0,8 g Endo-5'-Cyano-spiro [bicyclo [2.2.2] octan-2,3'-pyrrolidin] werden mit 20 ml 5 N Salzsäure 5 Stunden am Rückfluß gekocht. Nach Einengen zur Trockene wird mit Wasser aufgenommen, durch Zugabe von Amberlite® IRA 93 (OH-Form) auf einen pH-Wert von 5,7 gebracht, abfiltriert und eingeengt. Der Rückstand wird mit Methylenchlorid/Isopropylether verrieben. Man erhält 0,9 g der Titelverbindung als farblose Kristalle von Schmp. 236 °C.

$^1$H-NMR-Daten ($D_2O$) : 4,2 (x-Teil eines ABX-Systems, 1H) ; 3,2 (s, 2H) ; 2,5-1,5 (AB-Teil eines ABX-Systems, 2H) ; 1,5 (br. s, 12H) ppm.

Massenspektrum (m/e) : 209 ($M^+$, 0,8 %) ; 165 (13 %) ; 164 (M—COOH, 100 %) ; 87 (14 %), 69 (10 %).

Beispiel 3

Exo-Spiro-[bicyclo-[2.2.2] octan-2.3'-pyrrolidin]-5'-yl-carbonsäure

1,5 g Exo-5'-Cyano-Spiro-[bicyclo [2.2.2] octan-2.3'-pyrrolidin] werden mit 30 ml 5 N Salzsäure nach der im Beispiel 2 beschriebenen Verfahrensweise umgesetzt. Man erhält 1,68 g der Titelverbindung als farblose Kristalle vom Schmp. 242 °C

$^1$H-NMR-Daten ($D_2O$) : 4,15 (X-Teil eines ABX-Systems, 1H) ; 3,2 (AB-System, $J_{AB}$ = 15 Hz, 2H) ; 2,5-1,7 (AB-Teil eines ABX-Systems, 2H) ; 1,5 (br. s, 12H) ppm.

Massenspektrum (m/e) : 209 ($M^+$, 0,5 %) ; 165 (15 %) ; 164 (M—COOH, 100 %) ; 87 (10 %) ; 69 (8 %).

Beispiel 4

5'-Cyano-spiro [bicyclo [2.2.1] heptan-2,3'-pyrrolidin]

a) 2-Cyano-2-(1,3-dioxolan-2-yl)-methyl-bicyclo [2.2.1] heptan

7,2 g Norbornan-2-carbonitril werden mit 60 mmol Lithiumdiiospropylamid (aus 8,2 ml Diisopropylamin und 40 ml n-Butyllithium hergestellt) analog Beispiel 1a mit 7 g 2-Brommethyl-1,3-dioxolan umgesetzt. Nach Chromatographie an Kieselgel mit Essigester/Cyclohexan (1 : 6) als Laufmittel erhält man 8,0 g Titelverbindung als Öl

$^1$H-NMR-Daten ($CDCl_3$) : 4,95 (t, J = 6 Hz, 1H) ; 4,0-3,7 (m, 4H) ; 2,6-2,2 (m, 2H) 2,0-1,2 (m, 10H) ppm

b) 2-Aminoethyl-2-(1,3-dioxolan-2-yl) methyl-bicyclo [2.2.1] heptan

8 g 2-Cyanomethyl-2-(1,3-dioxolan-2-yl)-methyl-bicyclo [2.2.1] heptan werden mit 4,4 g Lithiumaluminiumhydrid analog Beispiel 1b reduziert. Man erhält 6,9 g farbloses Öl

$^1$H-NMR-Daten ($CDCl_3$) : 4,07 (t, J = 6 Hz, 1H) ; 4,0-3,6 (m, 4H) ; 2,6 (s, 2H) ; 2,4-0,7 (m, 14H) ppm

c) Spiro [bicyclo [2.2.1] heptan-2,3'-pyrrolin-Δ1'']

6,9 g 2-Aminoethyl-2-(1,3-dioxolan-2-yl)-methyl-bicyclo [2.2.1] heptan werden mit 125 ml 5 N Salzsäure nach Beispiel 1c cyclisiert. Man erhält 4,6 g der Titelverbindung als Gemisch aus Monomeren und Trimeren.

d) 5'-Cyano-Spiro [bicyclo [2.2.1] heptan-2,3'-pyrrolidin]

4,6 g Spiro [bicyclo [2.2.1] heptan-2,3'-pyrrolidin-Δ3''] werden mit 3,85 g Kaliumcyanid und 42,8 ml 2 N Salzsäure analog der in Beispiel 1d beschriebenen Verfahrensweise umgesetzt. Man erhält 4,4 g der Titelverbindung als Isomerengemisch.

$^1$H-NMR-Daten ($CDCl_3$) : 3,98 (X-Teil eines ABX-Systems) 1H) ; 3,2-1,0 (m, restliche H) ppm.

Beispiel 5

Spiro [bicyclo [2.2.1] heptan-2,3'-pyrrolidin]-5'-yl-carbonsäure

4,4 g 5'-Cyano-spiro [bicyclo [2.2.1] heptan -2,3'-pyrrolidin] werden mit 120 ml 5 N Salzsäure nach der in Beispiel 2 beschriebenen Verfahrensweise hydrolysiert. Man erhält 4,35 g der Titelverbindung als farbloses Pulver.

$^1$H-NMR-Daten ($D_2O$) : 4,3-4,0 (m, 1H) ; 3,5-3,0 (m, AB-System, 2H) ; 2,5-1,0 (m, 12H) ppm.

Beispiel 6

Endo-Spiro-[bicyclo [2.2.2] octan-2,3'-pyrrolidin]-5'-yl-carbonsäurebenzylester-Hydrochlorid

0,9 g Endo-Spiro-[bicyclo [2.2.2] octan-2,3'-pyrrolidin]-5'-yl-carbonsäure werden zu einer bei —5 °C hergestellten Lösung von 0,9 ml Thionylchlorid in 9 ml Benzylalkohol gegeben. Nach 1 Stunde bei —5 °C und 18 Stunden bei Raumtemperatur wird mit Wasser verdünnt und mit Essigester extrahiert. Die Essigester-Phase wird über Magnesiumsulfat getrocknet und eingeengt. Der Benzylalkohol wird am Hochvakuum entfernt. Der ölige Rückstand wird mit Diisopropylether verrieben, wobei das Produkt kristallisiert, Ausbeute 0,92 g, Schmp. 139-142 °C

$^1$H-NMR-Daten ($CDCl_3$) : 7,3 (s, 5H) ; 5,25 (s, 2H) ; 4,52 (t, 1H) ; 3,4 (s, 2H) ; 2,5-1,1 (m, 14H) ppm.

Beispiel 7

Exo-Spiro [bicyclo [2.2.2] octan-2,3'-pyrrolidin]-5'-yl-carbonsäure-benzylester-Hydrochlorid

1,6 g Exo-Spiro-[bicyclo [2.2.2] octan-2,3'-pyrrolidin]-5'-yl-carbonsäure werden mit 1,6 ml Thionylchlorid und 16 ml Benzylalkohol analog der in Beispiel 6 beschriebenen Verfahrensweise verestert, Ausbeute 1,68 g, Schmp. 149-151 °C

$^1$H-NMR-Daten ($CDCl_3$) : 7,3 (s, 5H) ; 5,4-5,0 (AB-System, 2H) ; 4,55 (t, 1H) ; 3,35 (s, 2H) ; 2,4-1,2 (m, 14H) ppm.

Beispiel 8

Spiro [bicyclo [2.2.1] heptan-2,3'-pyrrolidin]-5'-yl-carbonsäure-benzylester-Hydrochlorid

4,3 g Spiro-[bicyclo [2.2.1] heptan-2,3'-pyrrolidin]-5'-yl-carbonsäure werden mit 43 ml Thionylchlorid und 43 ml Benzylalkohol analog der in Beispiel 6 beschriebenen Verfahrensweise verestert, Ausbeute 6,7 g,

$^1H$-NMR-Daten (DMSO-$d_6$) : = 7,4 (s, 5H) ; 5,2 (s, 2H) ; 4,6-4,2 (m, 1H) ; 3,5-3,0 (m, 2H) ; 2,4-1,0 (m, 12H) ppm.

Massenspektrum (m/e) : 285 ($M^+$, 0,1 %) ; 150 ($M^+$—$CO_2$Bzl, 100 %)

Beispiel 9

N-(1-S-Carbethoxy-3-phenyl-propyl)-S-alanyl-exo-spiro [bicyclo [2.2.2] octan-2,3′-pyrrolidin]-5′-yl-S-carbonsäurebenzylester (Diastereomer A9) und N-(1-S-Carbethoxy-3-phenyl-propyl)-S-alanyl-exo-spiro [bicyclo [2.2.2] octan-2,3′-pyrrolidin]-5′-yl-R-carbonsäurebenzylester (Diastereomere B9)

1,6 g des Benzylesters aus Beispiel 7 werden zusammen mit 1,39 g N-(1-S-Carbethoxy-3-phenyl-propyl)-S-alanin, 0,59 g 1-Hydroxybenzotriazol und 0,66 ml N-Ethylmorpholin in 15 ml absoluten Dimethylformamid gelöst. 1,08 g Dicyclohexylcarbodiimid werden zugesetzt und 2 Stunden bei Raumtemperatur gerührt. Der Niederschlag wird abfiltriert, das Filtrat mit Essigester verdünnt, je 1x mit 10-prozentiger Citronensäure-Lösung, 1N Natriumhydrogencarbonatlösung, Wasser und gesättigter Kochsalzlösung gewaschen, über Natriumsulfat getrocknet und eingeengt. Chromatographie des Rohprodukts an Kieselgel mit Essigester/Cyclohexan (1 : 2) als Laufmittel ergibt die beiden Titelverbindungen.

Diastereomer A9 : $R_f$-Wert 0,26 (Essigester/Cyclohexan 1 : 1)

$^1H$-NMR-Daten ($CDCl_3$) : = 7,3 (s, 5H) ; 7,15 (s, 5H) ; 5,15 (AB-System, 2H) ; 4,55 (X-Teil eines ABX-Systems, 1H) ; 4,2 (s, 2H) ; 3,7-3,1 (m, 3H) ; 2,9-2,4 (m, 2H) ; 2,3-1,0 (m, 18H) ; 1,2 (d + t, 6H) ppm.

Massenspektrum (m/c) : 560 ($M^+$, 1 %), 487 (4 %), 355 (3 %), 234 (100 %).

Diastereomer B9 : $R_f$-Wert 0,20 (Essigester/Cyclohexan 1 : 1)

$^1H$-NMR-Daten ($CDCl_3$) : = 7,3 (s, 5H) ; 7,15 (s, 5H) ; 5,2 (s, 2H) ; 4,6-4,0 (m, 4H) ; 3,8-3,0 (m, 4H) ; 2,9-1,0 (m, 18H) ; 1,2 (d + t, 6H) ppm.

Massenspektrum (m/e) : 560 ($M^+$, 1 %) ; 487 (4 %) ; 355 (3 %), 348 (5 %) 234 (100 %).

Beispiel 10

N-(1-S-Carbethoxy-3-phenyl-propyl)-S-alanyl-endo-spiro-[bicyclo [2.2.2] octan-2,3′-pyrrolidin]-5′-yl-(RS)-carbonsäurebenzylester

0,91 g des Benzylesters aus Beispiel 6 werden mit 0,8 g N-(1-S-Carbethoxy-3-phenyl-propyl)-S-alanin, 0,34 g 1-Hydroxybenzotriazol, 0,38 ml N-Ethylmorpholin und 0,62 g Dicyclohexylcarbodiimid nach der in Beispiel 9 beschriebenen Verfahrensweise umgesetzt. Chromatographie an Kieselgel mit Essigester/Cyclohexan (1 : 2) als Laufmittel ergibt 0,96 g der Titelverbindung als Diastereomerengemisch.

$^1H$-NMR-Daten ($CDCl_3$) : = 7,3 (s, 5H) ; 7,15 (s, 5H) ;

5,2 (s, 2H) ;
4,7-4,0 (m, 3H) ;
3,6-3,0 (m, 4H) ;
2,9-2,4 (m, 2H) ;
2,4-1,0 (m, 24H) ppm.

Beispiel 11

N-(1-S-Carbethoxy-3-phenyl-propyl)-S-alanyl-spiro [bicyclo-[2.2.1]-heptan-2,3'-pyrrolidin]-5'-yl-carbonsäurebenzylester

(Diastereomere A11 bis C11)

2,0 g des Benzylesters aus Beispiel 8 werden mit 1,83 g N-(1-S-Carbethoxy-3-phenyl-propyl)-S-alanin, 0,78 g 1-Hydroxybenzotriazol, 0,87 ml N-Ethylmorpholin sowie 1,43 g Dicyclohexylcarbodiimid nach der in Beispiel 9 beschriebenen Verfahrensweise umgesetzt. Durch Chromatographie an Kieselgel mit Essigester/Cyclohexan als Laufmittel können drei Fraktionen aufgetrennt werden.

Diastereomer A11 (0,28 g) $R_f$-Wert (Essigester/Cyclohexan 1 : 1) 0,36

$^1$H-NMR-Daten ($CDCl_3$) :

= 7,3 (s, 5H) ;
7,15 (s, 5H) ;
5,2 (AB-System, 2H) ;
4,6-3,9 (m, 4H) ;
3,6-3,0 (m, 4H) ;
2,9-2,6 (m, 2H) ;
2,4-1,0 (m, 22H) ppm.

Diastereomer B11 (0,89 g) $R_f$-Wert (Essigester/Cyclohexan 1 : 1) 0,32

$^1$H-NMR-Daten ($CDCl_3$) :

= 7,3 (s, 5H) ;
7,15 (s, 5H) ;
5,15 (AB-System, 2H) ;
4,55 (X-Teil eines ABX-Systems 2H) ;
4,2 (q, 2H) ;
4,4-4,0 (m, 1H) ;
3,7-3,0 (m, 4H) ;
2,9-2,5 (m, 2H) ;
2,4-1,0 (m, 22H) ppm.

Diastereomer C11 (0,86 g) $R_f$-Wert (Essigester/Cyclohexan 1 : 1) 0,29

$^1$H-NMR-Daten ($CDCl_3$) :

= 7,3 (s, 5H) ;
7,15 (s, 5H) ;
5,2 (s, 2H) ;
4,7-4,0 (m, 4H) ;
3,8-3,1 (m, 4H) ;
2,9-2,5 (m, 2H) ;
2,4-1,0 (m, 22H) ppm.

Beispiel 12

N-(1-S-Carbethoxy-3-phenyl-propyl)-S-alanyl-exo-spiro [bicyclo [2.2.2]-octan-2,3'-pyrrolidin]-5'-yl-S-carbonsäure-Hydrochlorid

553 mg des Diastereomer A9 aus Beispiel 9 werden in 50 ml Ethanol gelöst und nach Zugabe von 0,3 g Pd/C (10 %) bei Raumtemperatur und Normaldruck hydriert. Nach Filtration wird mit 2,5 N ethanolischer Salzsäure sauer gestellt, eingeengt und mit Isopropylether verrieben.

Man erhält 0,42 g der Titelverbindung vom Schmelzpunkt 128-130 °C (Zersetzung)

$^1$H-NMR-Daten (DMSO-$d_6$) :

= 7,25 (s, 5H) ;
4,6-3,0 (m, 7H) ;
3,0-2,0 (m, 2H) ;
2,0-1,0 (m, 24H) ppm.

IR-Daten (K Br) : 3400, 2930, 2860, 1740, 1650, 1500, 1220, 750, 700 $cm^{-1}$.

Beispiel 13

N-(1-S-Carbethoxy-3-phenyl-propyl)-S-alanyl-exo-spiro-[bicyclo [2.2.2] octan-2,3'-pyrrolidin]-5'-yl-R-carbonsäure Hydrochlorid

0,62 g des Diastereomers B9 aus Beispiel 9 werden nach der in Beispiel 12 beschriebenen Verfahrensweise hydriert. Man erhält 0,41 g der Titelverbindung vom Schmp. 120 °C (Zersetzung)

$^1$H-NMR-Daten ($DMSO-d_6$) : = 7,25 (s, 5H) ; 4,6-3,0 (m, 7H) ; 3,3-2,0 (m, 2H) ; 2,0-1,0 (m, 18H) ; 1,2 (d + t, 6H) ppm.

IR-Daten (K Br) : 3400, 2930, 2860, 1740, 1650, 1500, 1225, 750, 700 $cm^{-1}$.

Beispiel 14

N-(1-S-Carbethoxy-3-phenyl-propyl)-S-alanyl-endo-spiro-[bicyclo [2.2.2] octan-2,3'-pyrrolidin]-5'-yl-(RS)-carbonsäure Hydrochlorid

0,94 g der Verbindung aus Beispiel 10 werden nach dem in Beispiel 12 beschriebenen Verfahren hydriert. Man erhält 0,7 g der Titelverbindung vom Schmp. 186-189 °C (Zersetzung).

$^1$H-NMR-Daten ($DMSO-d_6$) : 7,2 (s, 5H) ; 4,6-3,1 (m, 7H) ; 3,0-2,0 (m, 2H) ; 2,0-1,0 (m, 18H) ; 1,2 (d + t, 6H) ppm.

IR-Daten (K Br) : 3420, 2930, 2860, 1740, 1653, 1545, 1500, 1208, 750, 705 $cm^{-1}$.

Massenspektrum (m/e) : 470 ($M^-$, 0,01 %) ; 452 ($M^-$—$H_2O$, 16 %), 348 (100 %), 302 (47 %), 263 (16 %)

Beispiel 15

Endo-Spiro [bicyclo [2.2.2] octan-2,3'-pyrrolidin]-5'-yl-carbonsäure-tert.-butylester Hydrochlorid

Zu einer auf —10 °C gekühlten Lösung von 1,3 g der Aminosäure aus Beispiel 2 werden in 12 ml Dioxan 1 ml konz. Schwefelsäure und 6 g Isobutylen gegeben. Nach Aufwärmen im Autoklaven auf 25° wird 20 Stunden bei dieser Temperatur gerührt. man gibt das Gemisch in eiskaltes 50 %iges Natriumhydroxid und extrahiert mit Methylenchlorid. Die vereinigten organischen Phasen werden mit Wasser gewaschen, mit Natriumsulfat getrocknet, der Rückstand in Ether gelöst und gasförmiger Chlorwasserstoff eingeleitet. Man erhält 0.95 g der Titelverbindung

$^1$H-NMR-Daten ($DMSO-d_6$) : = 4,0-3,5 (m, 1H) ; 3,2-2,6 (s, 2H) ; 2,0-1,1 (m, 14H) ; 1,3 (s, 9H) ppm.

Beispiel 16

Exo-Spiro [bicyclo [2.2.2] octan-2,3'-pyrrolidin]-5'-yl-carbonsäure-tert.-butylester Hydrochlorid

Hergestellt aus 0,7 g der Aminosäure aus Beispiel 3 nach der in Beispiel 15 beschriebenen Verfahrensweise. Man erhält 0,6 g der Titelverbindung.

$^1$H-NMR-Daten ($DMSO-d_6$) : = 3,9-3,5 (m, 1H) ; 3,2-2,6 (m, 1H) ; 2,0-1,1 (m, 14H) ; 1,3 (s, 9H) ppm.

Beispiel 17

Spiro [bicyclo [2.2.1] heptan-2,3'-pyrrolidin]-5'-yl-carbonsäure-tert.-butylester Hydrochlorid

Hergestellt aus 0,6 g der Aminosäure aus Beispiel 5 nach der in Beispiel 15 beschriebenen Verfahrensweise ; man erhält 0,52 g der Titelverbindung.

$^1$H-NMR-Daten ($DMSO-d_6$) : = 3,8-3,5 (m, 1H) ; 3,2-2,5 (m, 1H) ; 2,0-1,1 (m, 12H) ; 1,3 (s, 9H) ppm.

Beispiel 18

N-(1-S-(Carbethoxy-3-phenyl-propyl)-S-alanyl-endo-spiro-[bicyclo [2.2.2] octan-2,3'-pyrrolidin]-5'-yl-S-carbonsäure-tert.-butylester
(Diastereomer A18)

Analog der Verfahrensweise von Beispiel 9 erhält man aus 0,58 g des tert. Butylesters aus Beispiel 15, 0.57 g N-(1-S-Carbethoxy-3-phenyl-propyl)-S-alanin, 0,27 g N-Hydroxybenzotriazol sowie 0,41 g Dicyclohexylcarbodiimid unter Zusatz von 0,23 g N-Ethylmorpholin 0,28 g der Titelverbindung als farbloses Öl.

$^1$H-NMR-Daten ($CDCl_3$) : = 7,1 (s, 5H) ; 4,5-4,1 (m, 1H) ; 4,2 (q, J = 7 Hz, 2H) ; 3,9-1,9 (m, 22H) ; 1,3 (s, 9H) ; 1,2 (d + t, J = 7$H_2$, 6H) ppm.

Beispiel 19

N-(1-S-Carbethoxy-3-phenyl-propyl)-S-alanyl-endo-spiro-[bicyclo [2.2.2] octan-2,3'-pyrrolidin]-5'-yl-S-carbonsäure Hydrochlorid

0,28 g des tert. Butylesters aus Beispiel 18 werden in 1,5 ml Trifluoressigsäure gelöst und bei 0 °C 3 Stunden gerührt. Die Trifluorossigsäure wird im Vakuum abgedampft, Toluol zugesetzt und nochmals eingeengt. Nach Filtration über eine kurze Kieselgelsäule mit Methylenchlorid/Ethanol (10 : 1) als Laufmittel wird mit ethanolischer Salzsäure sauer gestellt und eingeengt. Nach Lösen in wenig Methylenchlorid wird die Titelverbindung mit Diisopropylether ausgefällt. Man erhält 0,21 g, identisch mit der Verbindung aus Beispiel 12.

Beispiel 20

N-(1-S-Carboxy-3-phenyl-propyl)-S-alanyl-exo-spiro-[bicyclo [2.2.2] octan-2,3'-pyrrolidin]-5'-yl-S-carbonsäure

Eine Lösung von 0,25 g der Verbindung aus Beispiel 12 in 2 ml Wasser wird mit zwei Äquivalenten Kaliumhydroxid und einem 10 %igen Überschuß 4n Kaliumhydroxidlösung versetzt. Nach 8-stündigem Rühren bei 20 bis 25 °C wird die Reaktionslösung mit 2n Salzsäure auf einen pH-Wert von 4 gestellt und im Vakuum eingeengt. Man nimmt den Rückstand in Essigester auf und filtriert das abgeschiedene Salz ab. Die Essigesterlösung wird eingeengt, der Rückstand mit Diisopropylether verrieben und abgesaugt.

$^1$H-NMR-Daten ($DMSO-d_6$) : = 7,1 (s, 5H) ; 4,4-4,0 (m, 1H) ; 3,9-1,3 (m, 22H) ; 1,2 (d, 3H) ppm.

Beispiel 21

N-(1-S-Carboxy-3-phenyl-propyl)-S-alanyl-endo-spiro-[bicyclo [2.2.2] octan-2,3'-pyrrolidin]-5'-yl-(RS)-carbonsäure

0,32 g der Verbindung aus Beispiel 14 werden nach den in Beispiel 20 beschriebenen Verfahren umgesetzt.

$^1$H-NMR-Daten ($DMSO-d_6$) : = 7,15 (s, 5H) ; 4,4-3,9 (m, 1H) ; 3,9-1,3 (m, 22H) ; 1,2 (d, 3H) ppm.

Beispiel 22

N-(1-S-Carbethoxy-3-phenyl-propyl)-S-alanyl-spiro-[bicyclo [2.2.1] heptan-2,3'-pyrrolidin]-5'-yl-carbonsäure-Hydrochlorid
(Diastereomere A22 bis C22)

Die Diastereomere A11 bis C11 aus Beispiel 11 werden nach der in Beispiel 12 beschreibenen Vorschrift hydriert. Man erhält jeweils die freien Carbonsäuren.

Diastereomer A22 0,17 g, F. 189 °C (Zers.)

$^1$H-NMR-Daten (DMSO-$d_6$) : = 7,2 (s, 5H) ; 4,6-3,0 (m, 7H) ; 3,0-2,0 (m, 2H) ; 2,1-1,0 (m, 22H) ppm.

Diastereomer B22 0,79 g, F. 126 °C (Zers.)

$^1$H-NMR-Daten (DMSO-$d_6$) : = 7,2 (s, 5H) ; 4,5-3,1 (m, 7H) ; 3,1-2,0 (m, 2H) ; 2,2-1,0 (m, 22H) ppm.

Diastereomer C22 0,63 g, F. 125 °C (Zers.)

$^1$H-NMR-Daten (DMSO-$d_6$) : = 7,15 (s, 5H) ; 4,5-3,1 (m, 7H) ; 3,0-2,0 (m, 2H) ; 2,2-1,0 (m, 22H) ppm.

Beispiel 23

N-(1-S-Carbethoxy-3-phenyl-3-oxo-propyl)-S-alanyl-exo-spiro-[bicyclo [2.2.2] octan-2,3'-pyrrolidin]-5'-yl-S-carbonsäure-tert. butylester

0,28 g des tert. Butylesters aus Beispiel 16 werden zusammen mit 0,14 g 1-Hydroxybenzotriazol, 0,29 g N-(1-S)Carbethoxy-3-phenyl-3-oxo-propyl)-S-alanin, 0,22 g Dicyclohexylcarbodiimid und 0,12 g N-Ethylmorpholin in 3 ml DMF gelöst und 3 Stunden bei 20 °C gerührt. Nach Verdünnen mit Essigester wird filtriert, zweimal mit Wasser gewaschen, getrocknet und eingeengt. Der Rückstand wird an Kieselgel mit Cyclohexan/Essigester (1 : 1) als Laufmittel chromatographiert ; man erhält 0,16 g der Titelverbindung.

$^1$H-NMR-Daten ($CDCl_3$) : = 8,2-7,1 (m, 5H) ; 4,7-4,1 (m, 1H) ; 4,1 (q, 2H) ; 3,9-1,3 (m, 20H) ; 1,3 (s, 9H) ; 1,2 (d + t, 6H) ppm.

Beispiel 24

N-(1-S-Carbethoxy-3-phenyl-3-oxo-propyl)-S-alanyl-endo-spiro-[bicyclo [2.2.2] octan-2,3'-pyrrolidin]-5'-yl-S-carbonsäure-tert.-butylester

0,28 g des tert. Butylesters aus Beispiel 15 werden nach der Verfahrensweise von Beispiel 23 umgesetzt. Man erhält 0,18 g der Titelverbindung.

$^1$H-NMR-Daten ($CDCl_3$) : = 8,2-7,2 (m, 5H) ; 4,7-4,1 (m, 1H) ; 4,15 (q, 2H) ; 3,9-1,2 (m, 20H) ; 1,3 (s, 9H) ; 1,2 (d + t, 6H) ppm.

Beispiel 25

N-(1-S-Carbethoxy-3-phenyl-3-oxo-propyl)-S-alanyl-spiro-[bicyclo [2.2.1] heptan-2,3'-pyrrolidin]-5'-yl-S-carbonsäure-tert.-butylester

0,27 g des tert.-Butylesters aus Beispiel 17 werden nach der Verfahrensweise von Beispiel 23 umgesetzt.

$^1$H-NMR-Daten ($CDCl_3$) : = 8,1-7,2 (m, 5H) ; 4,7-4,0 (m, 1H) ; 4,1 (q, 2H) ; 3,9-1,2 (m, 18H) ; 1,3 (s, 9H) ; 1,2 (d + t, 6H) ppm.

Beispiel 26

N-(1-S-Carbethoxy-3-phenyl-3-oxo-propyl)-S-alanyl-exo-spiro [bicyclo [2.2.2] octan-2,3'-pyrrolidin]-5'-yl-S-carbonsäure-Hydrochlorid

0,12 g der Verbindung aus Beispiel 23 werden analog der in Beispiel 19 beschriebenen Verfahrensweise zu 0,1 g der Titelverbindung umgesetzt.

$^1$H-NMR-Daten (DMSO-$d_6$) : = 8,2-7,1 (m, 5H) ; 4,7-4,1 (m, 1H) ; 4,1 (q, 2H) ; 3,9-1,3 (m, 20H) ; 1,2 (d + t, 6H) ppm.

Beispiel 27

N-(1-S-Carbethoxy-3-phenyl-3-oxo-propyl)-S-alanyl-endo-spiro [bicyclo [2.2.2] octan-2,3'-pyrrolidin]-5'-yl-S-carbonsäure-Hydrochlorid

0,14 g der Verbindung aus Beispiel 24 werden analog der in Beispiel 19 beschriebenen Verfahrensweise zu 0,11 g der Titelverbindung umgesetzt.

$^1$H-NMR-Daten (DMSO-$d_6$) : = 8,2-7,1 (m, 5H) ; 4,7-4,1 (m, 1H) ; 4,1 (q, 2H) ; 3,9-1,3 (m, 20H) ; 1,2 (d + t, 6H) ppm.

Beispiel 28

N-(1-S-Carbethoxy-3-phenyl-3-oxo-propyl)-S-alanyl-spiro-[bicyclo [2.2.1] heptan-2,3'-pyrrolidin]-5'-yl-S-carbonsäure-Hydrochlorid

0,23 g der Verbindung aus Beispiel 25 werden analog der in Beispiel 19 beschriebenen Verfahrensweise zu 0,19 g der Titelverbindung umgesetzt.

$^1$H-NMR-Daten (DMSO-$d_6$) : = 8,2-7,1 (m, 5H) ; 4,7-4,1 (m, 1H) ; 4,2 (q, 2H) ; 3,8-1,2 (m, 18H) ; 1,2 (d + t, 6H) ppm.

Beispiel 29

S-Alanyl-exo-spiro [bicyclo [2.2.2] octan-2,3'-pyrrolidin]-5'-yl-carbonsäure-tert.-butylester

a) N-Methylsulfonylethyloxycarbonyl (MSC)-S-alanyl-exo-spiro-[bicyclo [2.2.2] octan-2,3'-pyrrolidin]-5'-yl-carbonsäure-tert. butylester

Zu einer Lösung von 10 g MSC-Ala-OH in 50 ml Dimethylformamid werden 6,7 g 1-Hydroxybenzotriazol und 16,0 g Exo-Spiro [bicyclo [2.2.2] octan-2,3'-pyrrolidin]-5'-yl-carbonsäure-tert. butylester gegeben. Der pH-Wert wird mit N-Ethylmorpholin auf 8,0 eingestellt. Das Gemisch wird im Eisbad gekühlt und mit 10,5 g Dicyclohexylcarbodiimid versetzt. Man rührt 15 Stunden bei 20-25 °C. Der ausgefallene Harnstoff wird abgesaugt, das Filtrat im Vakuum eingeengt, und in Essigester aufgenommen. Die organische Phase wird mit nacheinander Kaliumhydrogensulfat-, Kaliumhydrogencarbonat- und Natriumchloridlösung gewaschen, getrocknet und eingedampft. Der Rückstand wird an Kieselgel mit Essigester/Cyclohexan 1 : 1 chromatographiert. Ausbeute : 10 g

$^1$H-NMR-Daten ($CDCl_3$) : = 4,8-3,8 (m, 2H) ;
3,8-3,1 (m, 8H) ;
3,0 (s, 3H) ;
2,9-1,2 (m, 14H) ;
1,4 (s, 9H) ;
1,2 (d, 3H) ppm.

b) S-Alanyl-exo-spiro [bicyclo [2.2.2] octan-2,3'-pyrrolidin]-5'-yl-carbonsäure-tert. butylester

2,0 g der Verbindung aus Beispiel 29a werden in 15 ml Methanol und 1,5 ml Wasser gelöst. Es wird mit 2n Natronlauge auf pH 13 gebracht und 2 Stunden bei Raumtemperatur gerührt. Danach wird mit 2n Salzsäure neutralisiert, das Methanol im Vakuum abgedampft, die wäßrige Phase mit Essigester extrahiert, die Essigesterlösung mit Wasser gewaschen, getrocknet und eingeengt. Der Rückstand wird über Kieselgel mit Essigester als Elutionsmittel filtriert. Ausbeute : 0,8 g

$^1$H-NMR-Daten ($CDCl_3$) : = 4,7-4,2 (m, 1H) ;
3,9-3,3 (m, 3H) ;
2,9-1,2 (m, 19H) ;
1,4 (s, 9H) ;
1,2 (d, 3H) ppm.

## Beispiel 30

N-(1-S-Carbethoxy-3-oxo-3-phenyl-propyl)-S-alanyl-exo-spiro [bicyclo [2.2.2] octan-2,3'-pyrrolidin]-5'-yl-carbonsäure-tert, butylester

5 m Mol der Verbindung aus Beispiel 29 b werden zusammen mit 5 m Mol 3-Benzoyl-acrylsäureethylester und 5 Tropfen Triethylamin in 50 ml wasserfreiem Ethanol gelöst und das Gemisch 24 Stunden bei 20 bis 25 °C gerührt. Es wird zur Trockene eingedampft und der Rückstand in Essigester aufgenommen. Nun wird mit Wasser gewaschen, getrocknet und eingedampft. Das Diastereomerengemisch wird an Kieselgel mit Essigester/Cyclohexan als Elutionsmittel chromatographiert. Die $^1$H-NMR-Daten stimmen mit den Daten der Verbindung aus Beispiel 23 überein.

## Beispiel 31

N-(1-S-Carbethoxy-3-phenyl-propyl)-S-alanyl-exo-spiro [bicyclo [2.2.2] octan-2,3'-pyrrolidin]-5'-yl-S-carbonsäure-tert. butylester

5 mmol der Verbindung aus Beispiel 29 b werden in 15 ml wasserfreiem Ethanol gelöst. Man stellt die Lösung mit ethanolischem Kaliumhydroxid auf pH 7,0 und gibt 0,7 g pulverisiertes Molekularsieb (4 Å) und anschließend 5 m Mol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25 °C wird die Lösung filtriert und das Lösungsmittel abdestilliert. Der Rückstand wird in Essigester/Wasser aufgenommen. Nach dem Eindampfen der Essigesterphasen wird der Rückstand an Kieselgel mit Essigester/Cyclohexan 1 : 4 chromatographiert.

Die $^1$H-NMR-Daten stimmen mit den Daten der Verbindung aus Beispiel 18 überein.

## Beispiel 32

N-(1-S-Carbethoxy-3-phenyl-3-oxo-propyl)-S-alanyl-exo-spiro [bicyclo [2.2.2] octan-2,3'-pyrrolidin]-5'-yl-S-carbonsäure-tert. butylester

10 mmol Acetophenon, 10 mmol Glyoxylsäureethylester und 10 mmol der Verbindung aus Beispiel 23 b werden in 30 ml Eisessig 36 Stunden auf 45 °C erhitzt. Nach dem Einengen im Vakuum wird mit Natriumcarbonatlösung neutral gestellt und mit Essigester extrahiert. Die Essigesterphase wird eingeengt und an Kieselgel mit Essigester/Cyclohexan 1 : 1 als Elutionsmittels chromatographiert.

Die NMR-Daten stimmen mit den Daten der Verbindung aus Beispiel 23 überein.

Beispiel 33

N-(1-S-Carbethoxy-3-R,S-hydroxy-3-phenyl-propyl)-S-alanyl-exo-spiro [bicyclo [2.2.2] octan-2,3'-pyrrolidin]-5'-yl-S-carbonsäure

0,5 g der Verbindung aus Beispiel 23 werden in 5 ml wäßrigem Ethanol gelöst und 0,1 g Natriumborhydrid zugegeben. Es wird 14 Stunden bei Raumtemperatur gerührt. Danach wird mit Essigester versetzt, die Essigesterlösung mit Wasser gewaschen, getrocknet und eingedampft. Das Rohrprodukt wird über Kieselgel mit Essigester/Methanol 9 : 1 als Elutionsmittel filtriert. Ausbeute : 0,3 g

$^1$H-NMR-Daten (DMSO-$d_6$) : = 7,3-6,9 (m, 5H) ; 5,4 (t, 1H) ; 4,7-4,2 (m, 1H) ; 3,9-1,3 (m, 20H) ; 1,3 (d + t, 6H) ppm.

**Patentansprüche** (für die Vertragsstaaten : BE, CH, DE, FR, GB, IT, LI, LU, NL, SE)

1. Verbindung der Formel I

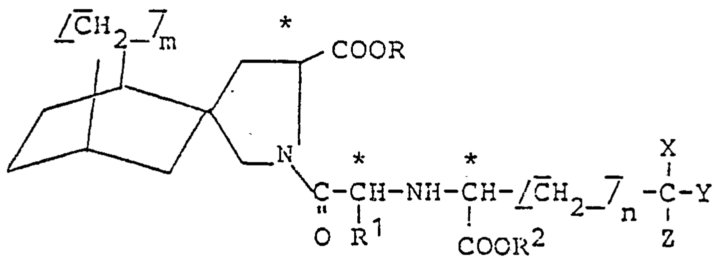

(I)

in welcher
m = 1 oder 2,
n = 0 oder 1,
R = Wasserstoff ($C_1$ bis $C_6$)-Alkyl oder Aralkyl mit 7 bis 9 C-Atomen,
$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$-$C_2$) alkyl, die beide wie vorstehend definiert im Arylrest substituiert sein können, ein mono- bzw. bicyclischer Heterocyclen-Rest mit 5 bis 7 bzw. 8 bis 10 Ringatomen, wobon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,
$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,
Y = Wasserstoff oder Hydroxy,
Z = Wasserstoff oder
Y und Z = zusammen Sauerstoff und
X = ($C_1$ bis $C_6$)-Alkyl, ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkylamino und/oder Methylendioxy mono-, di- oder trisubstituiert sein kann, oder 3-Indolyl bedeuten, sowie deren physiologisch unbedenkliche Salze.

2. Verbindung der Formel I gemäß Anspruch 1, dadurch gekennzeichnet, daß die mit einem Stern markierten C-Atome jeweils die S-Konfiguration aufweisen.

3. Verbindung der Formel I gemäß einem der Ansprüche 1 und 2, dadurch gekennzeichnet, daß
m = 1 oder 2,
n = 1,
R = Wasserstoff oder ($C_1$ bis $C_4$-Alkyl),
$R_1$ = Wasserstoff, ($C_1$ bis $C_3$)-Alkyl, ($C_2$ oder $C_3$)-Alkenyl, Benzyl, 4-Methoxybenzyl, 4-Ethoxybenzyl, Phenethyl, 4-Amino-butyl oder Benzoylmethyl,
$R^2$ = Wasserstoff, ($C_1$ bis $C_4$)-Alkyl oder Benzyl,
X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkylamino, Nitro und/oder Methylendioxy mono- oder disubstituiert oder im Falle von Methoxy, tri-substituiert sein kann, bedeuten.

4. Verbindung der Formel I gemäß Anspruch 1, dadurch gekennzeichnet, daß
m = 1 oder 2,
n = 1,
R = Wasserstoff,
$R^1$ = Methyl, 4-Methoxybenzyl oder 4-Ethoxybenzyl und
$R^2$ = Wasserstoff oder Ethyl bedeuten und die chiralen, mit einem Stern (*) markierten C-Atomen die S-Konfiguration besitzen.

5. Verfahren zur Herstellung der Verbindungen der Formel I gemäß einem der Ansprüche 1 bis 4, dadurch gekennzeichnet,

a) daß man eine Verbindung der Formel II

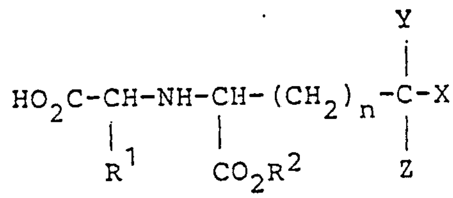

(II)

worin n, $R^1$, $R^2$, X, Y und Z die Bedeutung wie in Formel I haben mit einer Verbindung der Formel III

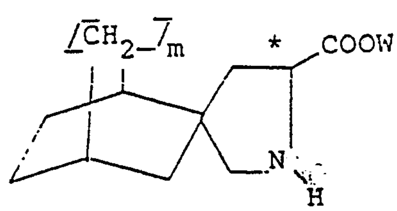

(III)

in welcher
m = 1 oder 2 und
W Wasserstoff oder einen sauer oder hydrogenolytisch abspaltbaren Rest bedeuten, umsetzt und anschließend gegebenenfalls W und/oder $R^1$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV

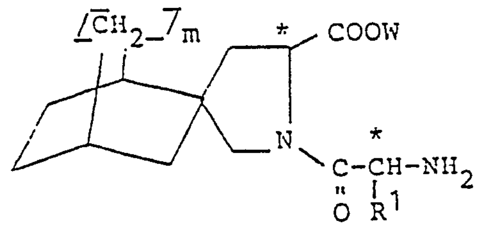

(IV)

in welcher m und $R^1$ die Bedeutung wie in Formel I und W die Bedeutung wie in Formel III besitzen, mit einer Verbindung der Formel V,

$$R^2O_2C—CH=CH—CO—X \qquad (V)$$

worin $R^2$ und X die Bedeutungen wie in Formel I haben, umgesetzt und anschließend gegebenenfalls W und/oder $R^2$ unter Bildung der freien Carboxygruppen abgespaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC—CO_2R^2 \quad (VI) \qquad X—CO—CH_3 \quad (VII)$$

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

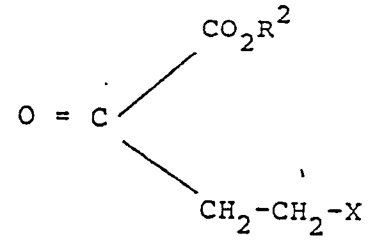

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert;

und die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt.

6. Verwendung einer Verbindung gemäß einem der Ansprüche 1 bis 4 zur Herstellung eines Arzneimittels.

7. Verbindung gemäß einem der Ansprüche 1 bis 4 zur Verwendung als Heilmittel.

8. Mittel, enthaltend eine Verbindung gemäß der Ansprüche 1 bis 4.

9. Mittel, enthaltend eine Verbindung gemäß einem der Ansprüche 1 bis 4 in Kombination mit einem Diuretikum.

10. Verwendung einer Verbindung gemäß einem der Ansprüche 1 bis 4, in Kombination mit einem Diuretikum zur Herstellung eines Arzneimittels.

11. Verbindung der Formel III

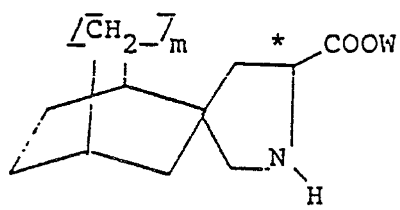

(III)

in welcher m = 1 oder 2 und W Wasserstoff oder einen sauer oder hydrogenolytisch abspaltbaren Rest bedeuten.

12. Verfahren zur Herstellung von Verbindungen der Formel III gemäß Anspruch 11, dadurch gekennzeichnet, daß man eine Verbindung der Formel XIV

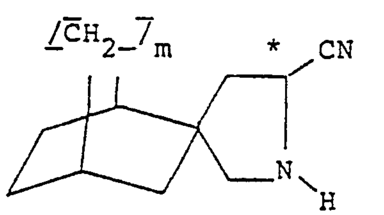

(XIV)

mit einer Mineralsäure oder mit einer starken Base verseift und die erhaltene Aminosäure (III/W = Wasserstoff) gegebenenfalls nach üblichen Methoden der Aminosäure-Chemie verestert.

**Patentansprüche** (für den Vertragsstaat AT)

1. Verfahren zur Herstellung der Verbindungen der Formel I

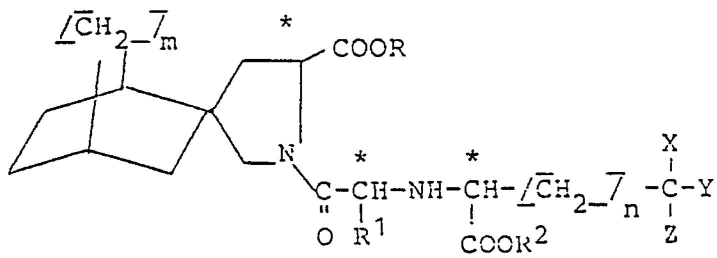

(I)

in welcher

m = 1 oder 2,

n = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$-$C_2$) alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,

$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkylamino und/oder Methylendioxy mono-, di-oder trisubstituiert sein kann, oder 3-Indolyl bedeuten, sowie deren physiologisch unbedenklichen Salzen, dadurch gekennzeichnet,

a) daß man eine Verbindung der Formel II

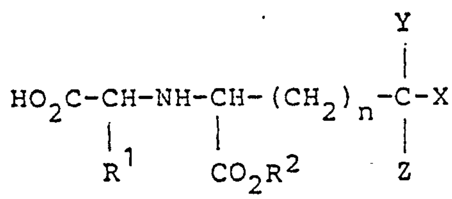

(II)

worin n, $R^1$, $R^2$, X, Y und Z die Bedeutung wie in Formel I haben mit einer Verbindung der Formel III

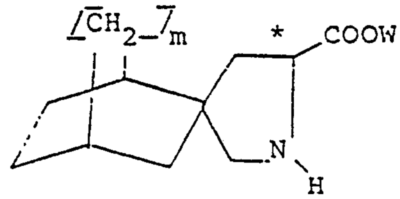

(III)

in welcher

m = 1 oder 2 und

W Wasserstoff oder einen sauer oder hydrogenolytisch abspaltbaren Rest bedeuten, umsetzt und anschließend gegebenenfalls W und/oder $R^1$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV

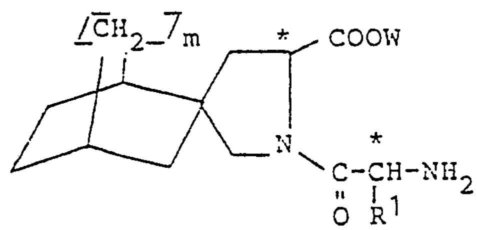

(IV)

in welcher m und $R^1$ die Bedeutung wie in Formel I und W die Bedeutung wie in Formel III besitzen, mit einer Verbindung der Formel V,

$$R^2O_2C—CH=CH—CO—X \qquad (V)$$

worin $R^2$ und X die Bedeutungen wie in Formel I haben, umgesetzt und anschließend gegebenenfalls W und/oder $R^2$ unter Bildung der Freien Carboxygruppen abgespaltet, oder

$b_2$) eine Verbindung der unter $b_1$) genannten Formel IV mit einer Verbindung der allgemeinen

Formel VI, worin $R^2$ die Bedeutung wie in Formel I hat, und mit einer Verbindung der allgemeinen Formel VII,

$$OHC—CO_2R^2 \quad (VI) \qquad X—CO—CH_3 \quad (VII)$$

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

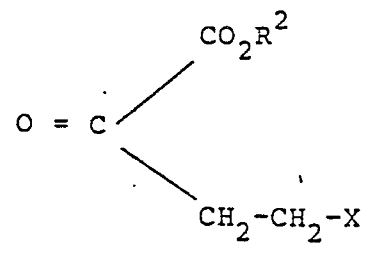

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert ;

und die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt.

2. Verfahren gemäß Anspruch 1, dadurch gekennzeichnet, daß in Formel I die mit einem Stern markierten C-Atome jeweils die S-Konfiguration aufweisen.

3. Verfahren gemäß einem der Ansprüche 1 und 2, dadurch gekennzeichnet, daß
m = 1 oder 2,
n = 1,
R = Wasserstoff oder ($C_1$ bis $C_4$-Alkyl),
$R_1$ = Wasserstoff, ($C_1$ bis $C_3$)-Alkyl, ($C_2$ oder $C_3$)-Alkenyl, Benzyl, 4-Methoxybenzyl, 4-Ethoxybenzyl, Phenethyl, 4-Amino-butyl oder Benzoylmethyl
$R_2$ = Wasserstoff, ($C_1$ bis $C_4$)-Alkyl oder Benzyl,
X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkylamino, Nitro und/oder Methylendioxy mono- oder disubstituiert oder im Falle von Methoxy, trisubstituiert sein kann, bedeuten.

4. Verfahren gemäß Anspruch 1, dadurch gekennzeichnet, daß
m = 1 oder 2,
n = 1,
R = Wasserstoff,
$R^1$ = Methyl, 4-Methoxybenzyl oder 3-Ethoxybenzyl und
$R^2$ = Wasserstoff oder Ethyl bedeuten und die chiralen, mit einem Stern (*) markierten C-Atomen die S-Konfiguration besitzen.

5. Verfahren gemäß einem der Ansprüche 1 bis 4 zur Herstellung einer Verbindung der Formel I zur Verwendung als Heilmittel.

6. Verfahren zur Herstellung eines Mittels, enthaltend eine Verbindung der Formel I gemäß einem der Ansprüche 1 bis 4, dadurch gekennzeichnet, daß man die in eine geeignete Darreichungsform bringt.

7. Verfahren gemäß Anspruch 6, dadurch gekennzeichnet, daß man ein Diuretikum zugibt.

8. Verfahren zur Herstellung von Verbindungen der Formel III

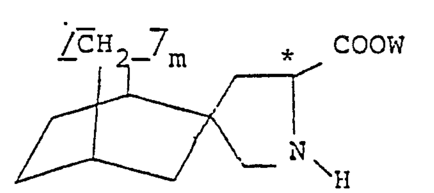

(III)

in welcher m = 1 oder 2 und W Wasserstoff oder einen sauer oder hydrogenolytisch abspaltbaren Rest bedeuten, dadurch gekennzeichnet, daß man eine Verbindung der Formel XIV

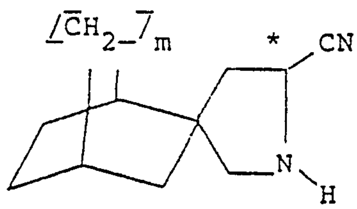

(XIV)

mit einer Mineralsäure oder mit einer starken Base verseift und die erhaltene Aminosäure (III/W = Wasserstoff) gegebenenfalls nach üblichen Methoden der Aminosäure-Chemie verestert.

**Claims** (for the Contracting States : BE, CH, DE, FR, GB, IT, LI, LU, NL, SE)

1. A compound of the formula I

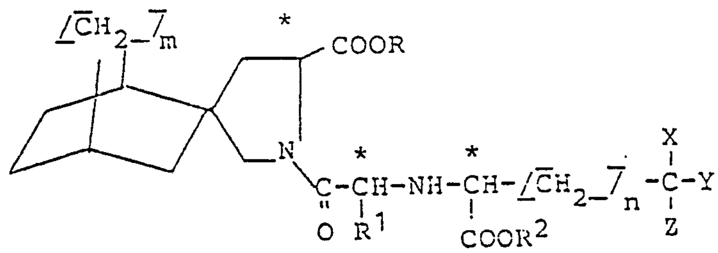

(I)

in which

m denotes 1 or 2,

n denotes 0 or 1,

R denotes hydrogen, ($C_1$ to $C_6$)-alkyl or aralkyl having 7 to 9 carbon atoms,

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $C_2$)-alkoxy or halogen, ($C_6$ to $C_{12}$)-aryl-($C_1$ to $C_4$)-alkyl or ($C_7$ to $C_{13}$)-aroyl-($C_1$ to $C_2$)-alkyl, both of which can be substituted in the aryl radical as defined above, a monocyclic or bicyclic heterocyclic radical having 5 to 7 or 8 to 10 ring atoms respectively, 1 to 2 ring atoms being sulfur or oxygen atoms and/or 1 to 4 ring atoms being nitrogen atoms, or an optionally protected side chain of a naturally occurring α-amino acid,

$R^2$ denotes hydrogen, ($C_1$ to $C_6$)-alkyl, ($C_2$ to $C_6$)-alkenyl or ($C_6$ to $C_{12}$)-aryl-($C_1$ to $C_4$)-alkyl,

Y denotes hydrogen or hydroxyl,

Z denotes hydrogen or

Y and Z together denote oxygen and

X denotes ($C_1$ to $C_6$)-alkyl, ($C_2$ to $C_6$)-alkenyl, ($C_5$ to $C_9$)-cycloalkyl, ($C_6$ to $C_{12}$)-aryl which can be monosubstituted, or trisubstituted by ($C_1$ to $C_4$)-alkyl, ($C_1$-$C_4$)-alkoxy, hydroxyl, halogen, nitro, amino ($C_1$ to $C_4$)-alkylamino, di-($C_1$-$C_4$)-alkylamino and/or methylenedioxy, or 3-indolyl,

and its physiologically acceptable salts.

2. A compound of the formula I as claimed in claim 1, wherein each of the carbon atoms marked with an asterisk has the S configuration.

3. A compound of the formula I as claimed in one of claims 1 and 2, wherein

m denotes 1 or 2,

n denotes 1,

R denotes hydrogen or ($C_1$ to $C_4$)-alkyl,

$R_1$ denotes hydrogen, ($C_1$ to $C_3$)-alkyl, ($C_2$ or $C_3$)-alkenyl, benzyl, 4-methoxybenzyl, 4-ethoxybenzyl, phenethyl, 4-aminobutyl or benzoylmethyl,

$R_2$ denotes hydrogen, ($C_1$ to $C_4$)-alkyl or benzyl and

X denotes cyclohexyl or phenyl which can be monosubstituted or disubstituted, or in the case of methoxy, trisubstituted, by ($C_1$ or $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy.

4. A compound of the formula I as claimed in claim 1, wherein

m denotes 1 or 2,

n denotes 1,

R denotes hydrogen,

$R^1$ denotes methyl, 4-methoxybenzyl or 4-ethoxybenzyl and

$R^2$ denotes hydrogen or ethyl, and the chiral carbon atoms marked with an asterisk (*) have the S configuration.

5. A process for the preparation of the compounds of the formula I as claimed in one of claims 1 to 4, which comprises

a) reacting a compound of the formula II

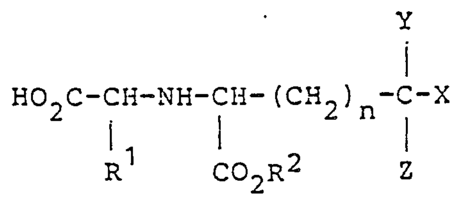

(II)

in which n, $R^1$, $R^2$, X, Y and Z have the same meaning as in formula I, with a compound of the formula III

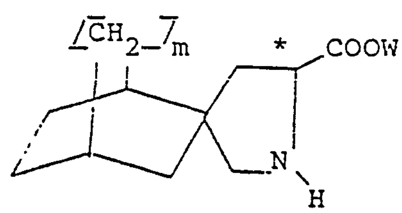

(III)

in which

m denotes 1 or 2 and

W denotes hydrogen or a radical which can be split off with acid or by hydrogenolysis, and then, where appropriate, splitting off W and/or $R^1$ with the formation of the free carboxyl groups, or

b) for the preparation of the compounds of the formula I in which Y and Z together denote oxygen,

$b_1$) reacting a compound of the formula IV

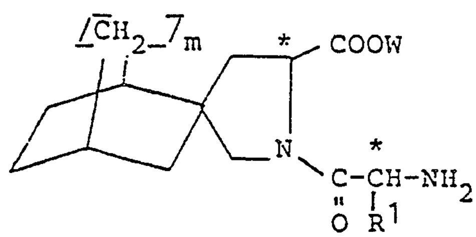

(IV)

in which m and $R^1$ have the same meaning as in formula I and W has ther same meaning as in formula II, with a compound of the formula V

$$R^2O_2C—CH=CH—CO—X \quad (V)$$

in which $R^2$ and X have the meanings as in formula I, and then, where appropriate, splitting off W and/or $R^2$ with the formation of the free carboxyl groups, or

$b_2$) reacting a compound of the formula IV mentioned under $b_1$) with a compound of the formula VI in which $R^2$ has the same meaning as in formula I, and with a compound of the general formula VII,

$$OHC—CO_2R^2 \quad (VI) \qquad X—CO—CH_3 \quad (VII)$$

in which X has the same meaning as in formula I, and then, where appropriate, splitting off W and/or $R^2$ with the formation of the free carboxyl groups, or

c) for the preparation of compounds of the formula I in which Y and Z each denote hydrogen, reacting a compound of the formula IV mentioned under $b_1$) with a compound of the formula VIII

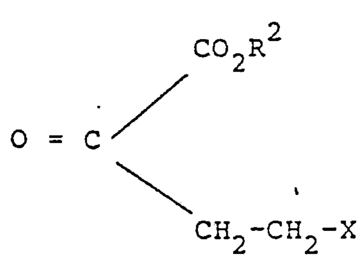

(VIII)

in which $R^2$ and X have the same meanings as in formula I, reducing the resulting Schiff's bases and then, where appropriate, splitting off W and/or $R^2$ with the formation of the free carboxyl groups, or

d) for the preparation of compounds of the formula I in which Y denotes hydroxyl and Z denotes hydrogen, reducing a compound of the formula I in which Y and Z together denote oxygen, with a complex boranate or borane-amine complex ;

and optionally converting the compounds of the formula I in which R represents hydrogen, obtained according to a) to d), into esters of the formula I in which R denotes ($C_1$ to $C_6$)-alkyl or ($C_7$ to $C_9$)-aralkyl.

6. The use of a compound as claimed in one of claims 1 to 4 for the preparation of a pharmaceutical.

7. A compound as claimed in one of claims 1 to 4 for use as a medicament.

8. An agent containing a compound as claimed in one of claims 1 to 4.

9. An agent containing a compound as claimed in one of claims 1 to 4 combined with a diuretic.

10. The use of a compound as claimed in one of claims 1 to 4 combined with a diuretic for the preparation of a pharmaceutical.

11. A compound of the formula III

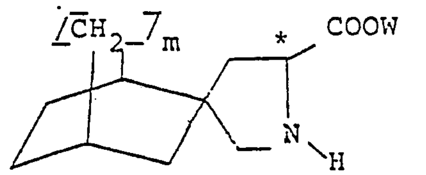

(III)

in which m denotes 1 or 2 and W denotes hydrogen or a radical which can be split off with acid or by hydrogenolysis.

12. A process for the preparation of compounds of the formula III, as claimed in claim 11, which comprises hydrolyzing a compound of the formula XIV

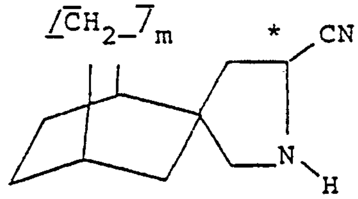

(XIV)

with a mineral acid or with a strong base, and esterifying the resulting amino acid (III/W = hydrogen), where appropriate, by methods customary in amino acid chemistry.

**Claims** (for the Contracting State AT)

1. A process for the preparation of the compounds of the formula I

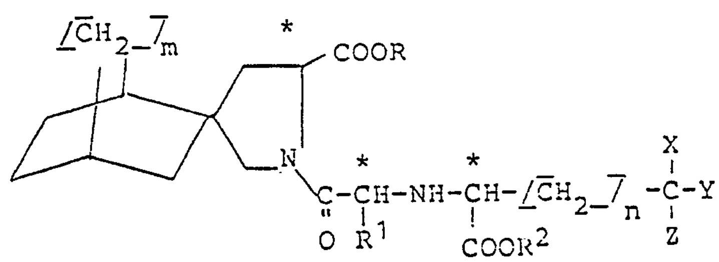

(I)

in which

m denotes 1 or 2,

n denotes 0 or 1,

R denotes hydrogen, ($C_1$ to $C_6$)-alkyl or aralkyl having 7 to 9 carbon atoms,

$R^1$ denotes hydrogen or ($C_1$ to $C_6$)-alkyl which can optionally be substituted by amino, ($C_1$ to $C_6$)-acylamino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $C_4$)-alkyl, ($C_1$ or $C_2$)-alkoxy or halogen, ($C_6$ to $C_{12}$)-aryl-(C· to $C_4$)-alkyl or ($C_7$ to $C_{13}$)-aroyl-($C_1$ to $C_2$)-alkyl, both of which can be substituted in the aryl radical as

defined above, a monocyclic or bicyclic heterocyclic radical having 5 to 7 or 8 to 10 ring atoms respectively, 1 to 2 ring atoms being sulfur or oxygen atoms and/or 1 to 4 ring atoms being nitrogen atoms, or an optionally protected side chain of a naturally occurring α-amino acid,

$R^2$ denotes hydrogen, ($C_1$ to $C_6$)-alkyl, ($C_2$ to $C_6$)-alkenyl or ($C_6$ to $C_{12}$)-aryl-($C_1$ to $C_4$)-alkyl,

Y denotes hydrogen or hydroxyl,

Z denotes hydrogen or

Y and Z together denote oxygen and

X denotes ($C_1$ to $C_6$)-alkyl, ($C_2$ to $C_6$)-alkenyl, ($C_5$ to $C_9$)-cycloalkyl, ($C_6$ to $C_{12}$)-aryl which can be monosubstituted, disubstituted or trisubstituted by ($C_1$ to $C_4$)-alkyl, ($C_1$-$C_4$)-alkoxy, hydroxyl, halogen, nitro, amino ($C_1$ to $C_4$)-alkylamino, di-($C_1$-$C_4$)-alkylamino and/or methylenedioxy, or 3-indolyl,

and its physiologically acceptable salts,

which comprises

a) reacting a compound of the formula II

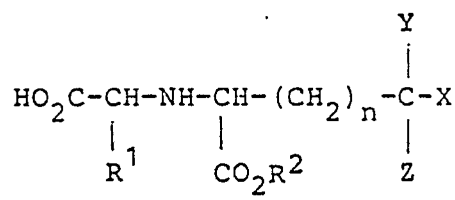

(II)

in which n, $R^1$, $R^2$, X, Y and Z have the same meaning as in formula I, with a compound of the formula III

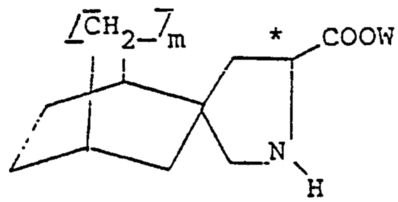

(III)

in which

m denotes 1 or 2 and

W denotes hydrogen or a radical which can be split off with acid or by hydrogenolysis, and then, where appropriate, splitting off W and/or $R^1$ with the formation of the free carboxyl groups, or

b) for the preparation of the compounds of the formula I in which Y and Z together denote oxygen,

$b_1$) reacting a compound of the formula IV

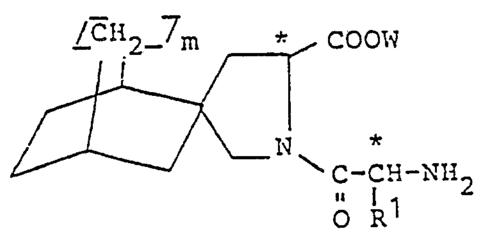

(IV)

in which m and $R^1$ have the same meaning as in formula I and W has the same meaning as in formula III, with a compound of the formula V

$$R^2O_2C—CH=CH—CO—X \quad (V)$$

in which $R^2$ and X have the meanings as in formula I, and then, where appropriate, splitting off W and/or $R^2$ with the formation of the free carboxyl groups, or $b_2$) reacting a compound of the formula IV mentioned under $b_1$) with a compound of the formula VI in which $R^2$ has the same meaning as in formula I, and with a compound of the general formula VII,

$$OHC—CO_2R^2 \quad (VI) \qquad X—CO—CH_2 \quad (VII)$$

in which X has the same meaning as in formula I, and then, where appropriate, splitting off W and/or $R^2$ with the formation of the free carboxyl groups, or

c) for the preparation of compounds of the formula I in which Y and Z each denote hydrogen, reacting a compound of the formula IV mentioned under $b_1$) with a compound of the formula VIII

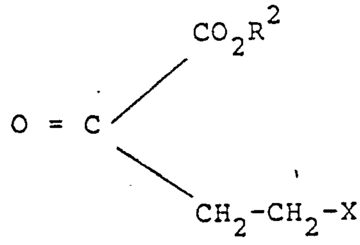

(VIII)

in which $R^2$ and X have the same meanings as in formula I, reducing the resulting Schiff's bases and then, where appropriate, splitting off W and/or $R^2$ with the formation of the free carboxyl groups, or

d) for the preparation of compounds of the formula I in which Y denotes hydroxyl and Z denotes hydrogen, reducing a compound of the formula I in which Y and Z together denote oxygen, with a complex boranate or borane-amine complex;

and optionally converting the compounds of the formula I in which R represents hydrogen, obtained according to a) to d), into esters of the formula I in which R denotes ($C_1$ to $C_6$)-alkyl or ($C_7$ to $C_9$)-aralkyl.

2. The process as claimed in claim 1, wherein each of the carbon atoms marked with an asterisk in formula I has the S configuration.

3. The process as claimed in one of claims 1 and 2, wherein
m denotes 1 or 2,
n denotes 1,
R denotes hydrogen or ($C_1$ to $C_4$)-alkyl,
$R_1$ denotes hydrogen, ($C_1$ to $C_3$)-alkyl, ($C_2$ or $C_3$)-alkenyl, benzyl, 4-methoxybenzyl, 4-ethoxybenzyl, phenethyl, 4-aminobutyl or benzoylmethyl,
$R_2$ denotes hydrogen, ($C_1$ to $C_4$)-alkyl or benzyl and
X denotes cyclohexyl or phenyl which can be monosubstituted or disubstituted, or in the case of methoxy, trisubstituted, by ($C_1$ or $C_2$)-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy.

4. The process as claimed in claim 1, wherein
m denotes 1 or 2,
n denotes 1,
R denotes hydrogen,
$R^1$ denotes methyl, 4-methoxybenzyl or 4-ethoxybenzyl and
$R^2$ denotes hydrogen or ethyl, and the chiral carbon atoms marked with an asterisk (*) have the S configuration.

5. The process as claimed in one of claims 1 to 4 for preparing a compound of the formula I for use as a medicament.

6. A process for the preparation of an agent containing a compound of the formula I as claimed in one of claims 1 to 4 which comprises converting it into a suitable administration form.

7. The process as claimed in claim 6, in which a diuretic is added.

8. A process for the preparation of compounds of the formula III

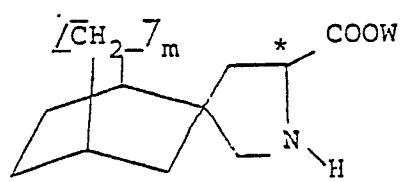

(III)

in which m denotes 1 or 2 and W denotes hydrogen or a radical which can be split off with acid or by hydrogenolysis, which comprises hydrolyzing a compound of the formula XIV

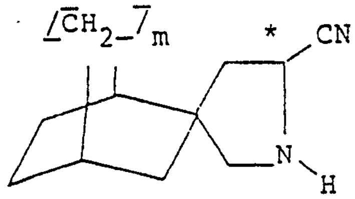

(XIV)

with a mineral acid or with a strong base, and esterifying the resulting amino acid (III/W = hydrogen), where appropriate, by methods customary in amino acid chemistry.

**Revendications** (pour les Etat contractants : BE, CH, DE, FR, GB, IT, LI, LU, NL, SE)

1. Composé de formule I

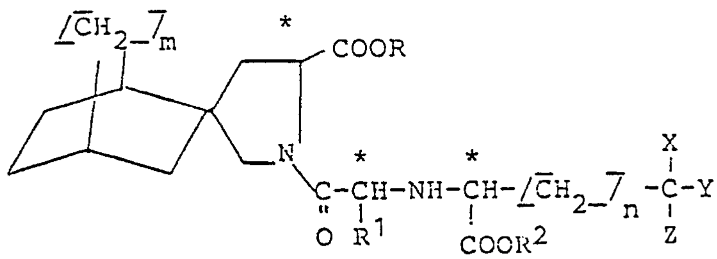

(I)

dans laquelle

m = 1 ou 2 ;

n = 0 ou 1 ;

R représente un atome d'hydrogène ou un groupe alkyle en $C_1$-$C_6$ ou aralkyle ayant de 7 à 9 atomes de carbone ;

$R^1$ représente un atome d'hydrogène ou un radical alkyle en $C_1$-$C_6$ qui peut éventuellement être substitué par un groupe amino, acyl($C_1$-$C_6$)-amino ou benzoylamino, un radical alcényle en $C_2$-$C_6$, cycloalkyle en $C_5$-$C_9$, cycloalcényle en $C_5$-$C_9$, cycloalkyl($C_5$-$C_7$)-alkyle($C_1$-$C_4$), aryle en $C_6$-$C_{12}$ ou aryle en $C_6$-$C_{12}$ partiellement hydrogéné, qui peut dans chaque cas être substitué par un groupe alkyle en $C_1$-$C_4$, alcoxy en $C_1$ ou $C_2$ ou par un halogène, un radical aryl($C_6$-$C_{12}$)-alkyle($C_1$-$C_4$) ou aroyl($C_7$-$C_{13}$)-alkyle($C_1$-$C_2$) qui peuvent être substitués chacun dans le fragment aryle comme défini plus haut, un radical hétérocyclique mono- ou bicyclique ayant de 5 à 7 ou de 8 à 10 atomes nucléaires, dont 1 ou 2 atomes nucléaires sont des atomes de soufre ou d'oxygène et/ou dont 1 à 4 atomes nucléaires sont des atomes d'azote, ou une chaîne latérale éventuellement protégée d'un α-aminoacide existant dans la nature ;

$R^2$ représente un atome d'hydrogène ou un radical alkyle en $C_1$-$C_6$, alcényl en $C_2$-$C_6$ ou aryl($C_6$-$C_{12}$)-alkyle($C_1$-$C_4$) ;

Y représente un atome d'hydrogène ou un groupe hydroxy ;

Z représente un atome d'hydrogène, ou

Y et Z représentent ensemble un atome d'oxygène ; et

X représente un radical alkyle en $C_1$-$C_6$, alcényle en $C_2$-$C_6$, cycloalkyle en $C_5$-$C_9$, aryle en $C_6$-$C_{12}$, qui peut être mono-, bi- ou trisubstitué par des atomes d'halogène ou par des groupes alkyle en $C_1$-$C_4$, alcoxy en $C_1$-$C_4$, hydroxy, nitro, amino, alkyl($C_1$-$C_4$)-amino, dialkyl($C_1$-$C_4$)-amino et/ou méthylènedioxy, ou le radical 3-indolyle ;

et sels physiologiquement acceptables de celui-ci.

2. Composé de formule I selon la revendication 1, caractérisé en ce que les atomes de carbone marqués par un astérisque présentent chacun la configuration S.

3. Composé de formule I selon l'une des revendications 1 et 2, caractérisé en ce que

m = 1 ou 2 ;

n = 1 ;

R représente un atome d'hydrogène ou un groupe alkyle en $C_1$-$C_4$ ;

$R^1$ représente un atome d'hydrogène ou un groupe alkyle en $C_1$-$C_3$, alcényle en $C_2$ ou $C_3$, benzyle, 4-méthoxybenzyle, 4-éthoxybenzyle, phénéthyle, 4-aminobutyle ou benzoylméthyle ;

$R^2$ représente un atome d'hydrogène ou un groupe alkyle en $C_1$-$C_4$ ou benzyle ; et

X représente un radical cyclohexyle ou phényle qui peut être mono- ou bisubstitué par des atomes de fluor, de chlore ou de brome, ou par des groupes alkyle en $C_1$ ou $C_2$, alcoxy en $C_1$ ou $C_2$, hydroxy, amino, alkyl($C_1$-$C_4$)-amino, dialkyl-($C_1$-$C_4$)-amino, nitro et/ou méthylènedioxy, ou trisubstitué dans le cas du groupe méthoxy.

4. Composé de formule I selon la revendication 1, caractérisé en ce que

m = 1 ou 2 ;

n = 1 ;

R représente un atome d'hydrogène ;

$R^1$ représente le groupe méthyle, 4-méthoxybenzyle ou 4-éthoxybenzyle ;

$R^2$ représente un atome d'hydrogène ou le groupe éthyle, et les atomes de carbone chiraux qui sont marqués par un astérique (*) ont la configuration S.

5. Procédé pour la préparation des composés de formule I selon l'une des revendications 1 à 4, caractérisé en ce que

a) on fait réagir un composé de formule II

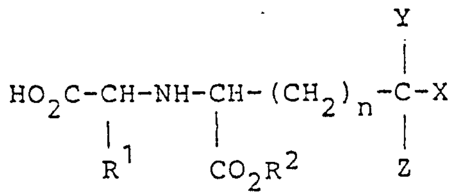

(II)

dans laquelle n, $R^1$, $R^2$, X, Y et Z ont les mêmes significations que dans la formule I, avec un composé de formule III

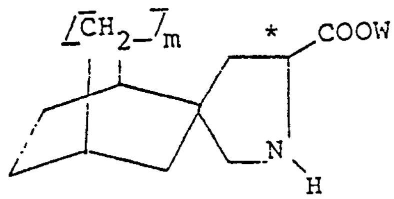

(III)

dans laquelle
m = 1 ou 2, et
W représente un atome d'hydrogène ou un radical séparable par traitement par un acide ou par hydrogénolyse et ensuite, éventuellement, on élimine W et/ou R1 avec formation des groupes carboxy libres ; ou

b) pour la préparation des composés de formule I dans lesquels Y et Z représentent ensemble un atome d'oxygène, $b_1$) on fait réagir un composé de formule IV

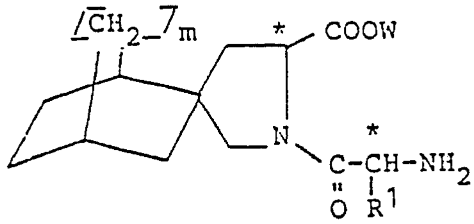

(IV)

dans laquelle m et $R^1$ ont les mêmes significations que dans la formule I, et W a la même signification que dans la formule III, avec un composé de formule V

$$R^2O_2C—CH=CH—CO—X \qquad (V)$$

dans laquelle $R^2$ et X ont les mêmes significations que dans la formule I, et ensuite, éventuellement, on élimine W et/ou $R^2$ avec formation des groupes carboxy libres ; ou

$b_2$) on fait réagir un composé de formule IV indiquée en $b_1$) avec un composé de formule générale VI

$$OHC—CO_2R^2 \qquad (VI)$$

dans laquelle $R^2$ a la même signification que dans la formule I, et avec un composé de formule générale VII

$$X—CO—CH_3 \qquad (VII)$$

dans laquelle X a la même signification que dans la formule I, et ensuite, éventuellement, on élimine W et/ou $R^2$ avec formation des groupes carboxy libres ; ou

c) pour la préparation de composés de formule I dans lesquels Y et Z représentent chacun un atome d'hydrogène, on fait réagir un composé de formule IV indiquée en $b_1$), avec un composé de formule VIII

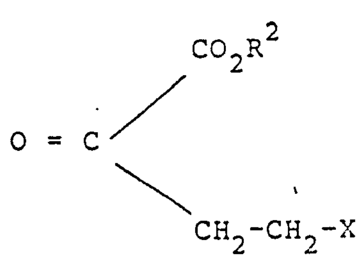

(VIII)

dans laquelle $R^2$ et X ont les mêmes significations que dans la formule I, on réduit les bases de Schiff obtenues et ensuite, éventuellement, on élimine W et/ou $R^2$ avec formation des groupes carboxy libres ; ou

d) pour la préparation de composés de formule I dans lesquels Y = hydroxy et Z = hydrogène, on réduit au moyen d'un boranate complexe ou d'un complexe borane-amine un composé de formule I dans lequel Y et Z représentent ensemble un atome d'oxygène ;
et on transforme éventuellement les composés de formule I dans lesquels R représente un atome d'hydrogène, obtenus selon a) à d), en esters de formule I, dans lesquels R représente un groupe alkyle en $C_1$-$C_6$ ou aralkyle en $C_7$-$C_9$.

6. Utilisation d'un composé selon l'une des revendications 1 à 4, pour la préparation d'un médicament.

7. Composé selon l'une des revendications 1 à 4, à utiliser en tant que médicament.

8. Agent contenant un composé selon l'une des revendications 1 à 4.

9. Agent contenant un composé selon l'une des revendications 1 à 4, en association avec un diurétique.

10. Utilisation d'un composé selon l'une des revendications 1 à 4, en association avec un diurétique pour la préparation d'un médicament.

11. Composé de formule III

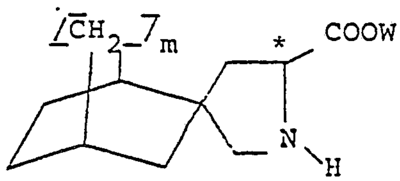

(III)

dans laquelle m = 1 ou 2, et W représente un atome d'hydrogène ou un radical séparable par traitement par un acide ou par hydrogénolyse.

12. Procédé pour la préparation de composés de formule III selon la revendication 11, caractérisé en ce que l'on saponifie avec un acide minéral ou avec une base forte un composé de formule XIV

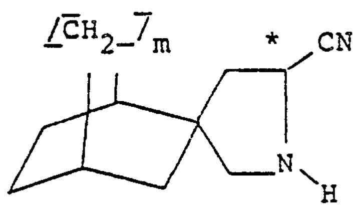

(XIV)

et, éventuellement, on estérifie l'aminoacide obtenu (III/W = hydrogène) selon des méthodes usuelles de la chimie des aminoacides.

**Revendications** (pour l'Etat contractant AT)

1. Procédé pour la préparation des composés de formule I

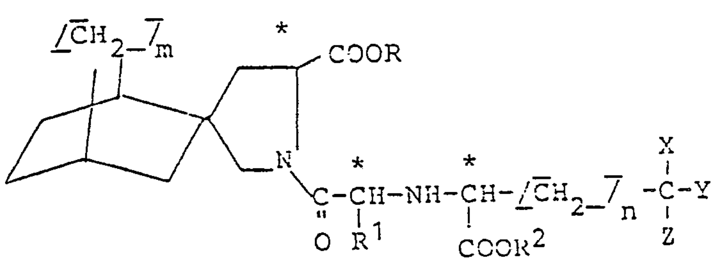

(I)

dans laquelle
m = 1 ou 2 ;
n = 0 ou 1 ;
R représente un atome d'hydrogène ou un groupe alkyle en $C_1$-$C_6$ ou aralkyle ayant de 7 à 9 atomes de carbone ;

$R^1$ représente un atome d'hydrogène ou un radical alkyle en $C_1$-$C_6$ qui peut éventuellement être substitué par un groupe amino, acyl($C_1$-$C_6$)-amino ou benzoylamino, un radical alcényle en $C_2$-$C_6$)-amino ou benzoylamino, un radical alcényle en $C_2$-$C_6$, cycloalkyle en $C_5$-$C_9$, cycloalcényle en $C_5$-$C_9$, cycloalkyl($C_5$-$C_7$)-alkyle($C_1$-$C_4$), aryle en $C_6$-$C_{12}$ ou aryle en $C_6$-$C_{12}$ partiellement hydrogéné, qui peut dans chaque cas être substitué par un groupe alkyle en $C_1$-$C_4$, alcoxy en $C_1$ ou $C_2$ ou par un halogène, un radical aryl($C_6$-$C_{12}$)-alkyle($C_1$-$C_4$) ou aroyl($C_7$-$C_{13}$)-alkyle($C_1$-$C_2$) qui peuvent être substitués chacun dans le fragment aryle comme défini plus haut, un radical hétérocyclique mono- ou bicyclique ayant de 5 à 7 ou de 8 à 10 atomes nucléaires, dont 1 ou 2 atomes nuclaires sont des atomes de soufre ou d'oxygène et/ou dont 1 à 4 atomes nucléaires sont des atomes d'azote, ou une chaîne latérale éventuellement protégée d'un α-aminoacide existant dans la nature ;

$R^2$ représente un atome d'hydrogène ou un radical alkyle en $C_1$-$C_6$, alcényle en $C_2$-$C_6$ ou aryl($C_6$-$C_{12}$)-alkyle($C_1$-$C_4$) ;

Y représente un atome d'hydrogène ou un groupe hydroxy ;

Z représente un atome d'hydrogène, ou

Y et Z représentent ensemble un atome d'oxygène ; et

X représente un radical alkyle en $C_1$-$C_6$, alcényle en $C_2$-$C_6$, cycloalkyle en $C_5$-$C_9$, aryle en $C_6$-$C_{12}$, qui peut être mono-, bi- ou trisubstitué par des atomes d'halogène ou par des groupes alkyle en $C_1$-$C_4$, alcoxy en $C_1$-$C_4$, hydroxy, nitro, amino, alkyl($C_1$-$C_4$)-amino, dialkyl($C_1$-$C_4$)-amino et/ou méthylènedioxy, ou le radical 3-indolyle ;

et de leurs sels physiologiquement acceptables, caractérisé en ce que

a) on fait réagir un composé de formule II

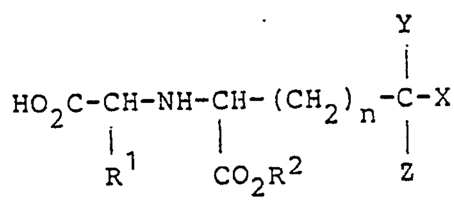

(II)

dans laquelle n, $R^1$, $R^2$, X, Y et Z ont les mêmes significations que dans la formule I, avec un composé de formule III

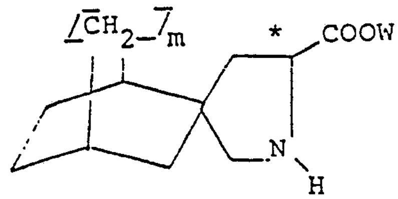

(III)

dans laquelle

m = 1 ou 2, et

W représente un atome d'hydrogène ou un radical séparable par traitement par un acide ou par hydrogénolyse,

et ensuite, éventuellement, on élimine W et/ou $R^1$ avec formation des groupes carboxy libres ; ou

b) pour la préparation des composés de formule I dans lesquels Y et Z représentent ensemble un atome d'oxygène,

$b_1$) on fait réagir un composé de formule IV

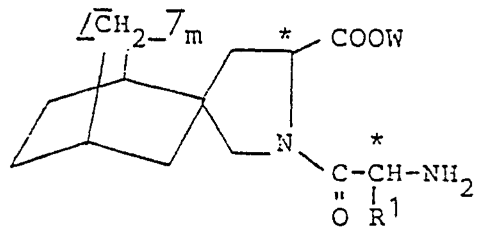

(IV)

dans laquelle m et $R^1$ ont les mêmes significations que dans la formule I, et W a la même signification que dans la formule III, avec un composé de formule V

$$R^2O_2C—CH=CH—CO—X \qquad (V)$$

dans laquelle $R^2$ et X ont les mêmes significations que dans la formule I, et ensuite, éventuellement, on élimine W et/ou $R^2$ avec formation des groupes carboxy libres ; ou

$b_2$) on fait réagir un composé de formule IV indiquée en $b_1$) avec un composé de formule générale VI

$$OHC—CO_2R^2 \qquad (VI)$$

dans laquelle $R^2$ a la même signification que dans la formule I, et avec un composé de formule générale VII

$$X—CO—CH_3 \qquad (VII)$$

dans laquelle X a la même signification que dans la formule I, et ensuite, éventuellement, on élimine W et/ou $R^2$ avec formation des groupes carboxy libres ; ou

c) pour la préparation de composés de formule I dans lesquels Y et Z représentent chacun un atome d'hydrogène, on fait réagir un composé de formule IV indiquée en $b_1$), avec un composé de formule VIII

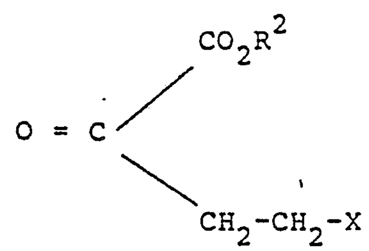

(VIII)

dans laquelle $R^2$ et X ont les mêmes significations que dans la formule I, on réduit les bases de Schiff obtenues et ensuite, éventuellement, on élimine W et/ou $R^2$ avec formation des groupes carboxy libres ; ou

d) pour la préparation de composés de formule I dans lesquels Y = hydroxy et Z = hydrogène, on réduit au moyen d'un boranate complexe ou d'un complexe borane-amine un composé de formule I dans lequel Y et Z représentent ensemble un atome d'oxygène ;

et on transforme éventuellement les composés de formule I dans lesquels R représente un atome d'hydrogène, obtenus selon a) à d), en esters de formule I, dans lesquesl R représente un groupe alkyle en $C_1$-$C_6$ ou aralkyle en $C_7$-$C_9$.

2. Procédé selon la revendication 1, caractérisé en ce que, dans la formulee I, les atomes de carbone marqués par un astérisque présentent chacun la configuration S.

3. Procédé selon l'une des revendications 1 et 2, caractérisé en ce que

m = 1 ou 2 ;

n = 1 ;

R représente un atome d'hydrogène ou un groupe alkyle en $C_1$-$C_4$ ;

$R^1$ représente un atome d'hydrogène ou un groupe alkyle en $C_1$-$C_3$, alcényle en $C_2$ ou $C_3$, benzyle, 4-méthoxybenzyle, 4-éthoxybenzyle, phénéthyle, 4-aminobutyle ou benzoylméthyle ;

$R^2$ représente un atome d'hydrogène ou un groupe alkyle en $C_1$-$C_4$ ou benzyle ;

X représente un radical cyclohexyle ou phényle qui peut être mono- ou bisubstitué par des atomes de fluor, de chlore ou de brome, ou par des groupes alkyle en $C_1$ ou $C_2$, alcoxy en $C_1$ ou $C_2$, hydroxy, amino, alkyl($C_1$-$C_4$)-amino, dialkyl($C_1$-$C_4$)-amino, nitro et/ou méthylènedioxy, ou trisubstitué dans le cas du groupe méthoxy.

4. Procédé selon la revendication 1, caractérisé en ce que

m = 1 ou 2 ;

n = 1 ;

R représente un atome d'hydrogène ;

$R^1$ représente le groupe méthyle, 4-méthoxybenzyle ou 4-éthoxybenzyle ; et

$R^2$ représente un atome d'hydrogène ou le groupe éthyle ; et les atomes de carbone chiraux qui sont marqués par un astérisque (*) ont la configuration S.

5. Procédé selon l'une des revendications 1 à 4, pour la préparation d'un composé de formule I à utiliser en tant que médicament.

6. Procédé pour la préparation d'un agent contenant un composé de formule I selon l'une des revendications 1 à 4, caractérisé en ce qu'on le met sous une forme pharmaceutique appropriée.

7. Procédé selon la revendication 6, caractérisé en ce que l'on ajoute un diurétique.

8. Procédé pour la préparation de composés de formule III

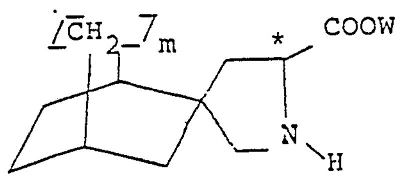

(III)

dans laquelle m = 1 ou 2, et W représente un atome d'hydrogène ou un radical séparable par traitement par un acide ou par hydrogénolyse, caractérisé en ce que l'on saponifie avec un acide minéral ou avec une base forte un composé de formule XIV

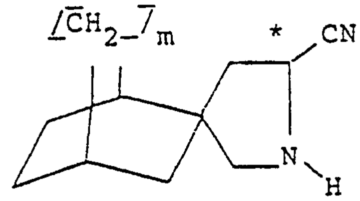

(XIV)

et, éventuellement, on estérifie l'aminoacide obtenu (III/W = hydrogène) selon des méthodes usuelles de la chimie des aminoacides.